# EUROPEAN PATENT APPLICATION

(11) **EP 4 509 134 A1**
(43) Date of publication of application: **19.02.2025**
(21) Application number: 23788605.6
(22) Date of filing: 12.04.2023
(51) Int. Cl.: A61K 38/20, A61P 29/00, A61P 35/00, C07K 14/54

(54) **COMPOSITION INCLUDING IL-32 THETA MUTANT AND USE THEREOF**

(30) Priority: 15.04.2022 KR 20220046961
(71) Applicant: Konkuk University Industrial Cooperation Corp, Seoul 05029 (KR)
(72) Inventor: YOON, Do-Young, Yangpyeong-gun, Gyeonggi-do 12505 (KR); PARK, Hyo Min, Namyangju-si, Gyeonggi-do 12233 (KR); PARK, Jaeyoung, Seoul 01677 (KR)
(74) Representative: Dolphin, Kirsty Mairi
(86) International application number: PCT/KR2023/004968
(87) International publication number: WO 2023/200256

(57) **Abstract**

The present invention relates to a pharmaceutical composition including an IL-32θ mutant for the prevention or treatment of inflammatory diseases and/or cancer and a use thereof.

The interleukin-32θ mutant (IL-32θ mutant) of the present invention can inhibit inflammatory cytokines and chemokines, inflammatory mediators and the metastasis and proliferation of cancer through intracellular pathways, and can suppress the expression of Intracellular Adhesion Molecule-1 (ICAM-1) and Vascular Cell Adhesion Molecule-1 (VCAM-1) in Human Umbilical Vein Endothelial Cells (HUVECs), and thus can be used for preventing and treating inflammatory diseases such as atherosclerosis through the inhibition of monocyte-endothelial cell adhesion. The mutant can also be utilized as a research material for various cancers and/or inflammatory diseases.

## Description

### Technical Field

This application claims priority to and the benefit of Korean Patent Application No. 10-2022-0046961, filed on April 15, 2022, the disclosure of which is incorporated herein by reference in its entirety.

The present invention relates to a pharmaceutical composition for preventing or treating cancer and/or an inflammatory disease, including IL-32θ mutant, and use thereof.

The present invention was completed under Project Number 2021R1A2C3014577 (1711129001) with the support of the Ministry of Science and ICT of the Republic of Korea.

### Background Art

IL-32, which was previously known as NK4, is expressed on activated human T cells and NK cells after stimulation with mitogens or IL-2. Previous studies have shown that NK4 induces pro-inflammatory cytokines such as IL-8, TNF-α and MIP-2 in several immune cells through classical cytokine signaling pathways, but lacks sequence homology with other cytokines. Therefore, its name was changed to IL-32, which is a member of the interleukin family. Numerous studies have reported that IL-32 is associated with cancer growth and development, viral infections and chronic inflammatory diseases such as Crohn's disease, inflammatory bowel disease and rheumatoid arthritis, indicating the ability of IL-32 to function as a cytokine. The IL-32 gene includes 8 exons and has several splice variants with different structures. IL-32α, IL-32β, IL-32γ and IL-32δ were first discovered in NK cells, and IL-32γ has the longest sequence among IL-32 isoforms. IL-32ε, IL-32ζ, IL-32η, IL-32θ and IL-32sm were additionally identified, and a total of 9 isoforms were reported. Early studies on IL-32 suggest that it is a proinflammatory cytokine. However, further study of individual isoforms shows that they play different roles.

IL-32θ, which is one of the IL-32 isoforms, is the only isoform other than IL-32sm with exon 6 deleted. Previous studies on IL-32θ were limited to overexpression, and its specific receptor was not clearly identified. IL-32α and IL-32β have been reported to bind to integrin αVβ3 and integrin αVβ6.

Breast cancer is one of the most prevalent cancers worldwide (11.7% of all types) and a leading cause of death (6.9% of all deaths). It is the most frequently diagnosed cancer in women (24.5%) and was one of the leading causes of cancer-related deaths (15.5%) in 2020. Whereas the global incidence of breast cancer increased rapidly in the 1980s and 1990s, the incidence decreased in the early 2000s due to advances in menopausal hormone therapy. However, breast cancer subtypes are identified by the absence or presence of three types of receptors: estrogen receptor, progesterone receptor and human epidermal growth factor receptor 2. Because these receptors are expressed on the surface of breast cancer cells, triple-negative breast cancer (TNBC) reduces the success of hormonal therapy, indicating the need to develop alternative treatment strategies. Cancer immunology has emerged as one of the 50 most exciting research topics in cancer treatment. The role of several cytokines, chemokines and inflammatory mediators in cancer progression has been studied in relation to inflammation, angiogenesis, anti-apoptosis, migration and proliferation. In particular, tumor-associated macrophages (TAMs), which are one of the most abundant cells in the tumor microenvironment, induce angiogenesis, metastasis and proliferation. Tumor-infiltrating immune cells stimulate several inflammatory molecules in cancer. Since its discovery as an inflammatory cytokine in 2005, several isoforms of IL-32 have been shown to play different non-canonical roles in various cancers and inflammatory and immune diseases, such as allergies, obesity and brain diseases. In particular, IL-32θ, which is a new isoform, has been reported to significantly inhibit proinflammatory cytokines and chemokines as well as epithelial-mesenchymal transition (EMT) markers. Although numerous studies on IL-32 have been conducted worldwide, the function of IL-32 isoforms in breast cancer is still unknown.

Cardiovascular disease (CVD) is a leading cause of death worldwide, causing 16.7 million deaths each year, and it is predicted that 23.6 million people will die from CVD each year by 2030. Coronary artery disease (CAD) and cerebrovascular disease, which are the most common forms of coronary artery disease, are recognized as a social problem beyond individual health. Atherosclerosis, which is a chronic inflammatory disease, was identified as the root cause. Atherosclerosis is caused by inflammation and damage to endothelial cells and accumulation of oxidized low-density lipoprotein (ox-LDL), and is characterized by the fibrosis of surrounding tissues and the formation of plaques composed of vascular endothelial cells, smooth muscle cells and foam cells. It is known as lipid-laden macrophages. Plaque development interferes with blood flow in blood vessels in various parts of the body, resulting in fatal diseases. The most important step in the early stages of atherosclerotic lesion formation is trans-endothelial migration (TEM), which refers to immune cells such as monocytes and leukocytes attaching to endothelial cells, infiltrating between endothelial cells and migrating into tissues. Vascular endothelial damage and the resulting inflammatory response increase the expression of cell adhesion molecules, such as intracellular adhesion molecule-1 (ICAM-1) and vascular cell adhesion molecule-1 (VCAM-1), on endothelial cells by inflammatory cytokines, such as IL-1β and TNF-α. Free-floating and blood monocytes adjacent to the vascular endothelium are captured by ICAM-1 and VCAM-1 expressed on endothelial cells. These adhesion molecules promote monocyte-endothelial adhesion through their respective interactions with lymphocyte function-related antigen 1 (LFA-1) and very late antigen-4 (VLA-4) expressed on monocytes, thereby ultimately promoting TEM. Monocytes passing through the vascular endothelium differentiate into macrophages, and the macrophages generate foam cells through the uptake of ox-LDL, thereby ultimately leading to the pathogenesis of atherosclerosis and plaque development. Previous studies have reported that IL-32β and IL-32γ act as proinflammatory cytokines involved in the upregulation of ICAM-1 and VCAM-1. However, the function of IL-32θ or its mutation in CVD is still unknown.

### [Related Art Documents]

### [Patent Documents]

(Patent Document 1) KR 10-2019-0118813 (September 26, 2019)

### Disclosure

### Technical Problem

Accordingly, the inventors of the present invention have made diligent efforts to provide a cytokine that can effectively suppress cancer and/or inflammatory diseases. As a result, the present invention was completed by confirming that the IL-32θ mutant of the present invention can suppress an inflammatory response and suppress cancer and/or inflammatory disease by immune action.

Therefore, an object of the present invention is to provide a pharmaceutical composition for preventing or treating cancer and/or an inflammatory disease, including an interleukin-32θ mutant (IL-32θ mutant), as well as use thereof.

### Technical Solution

The present invention relates to a pharmaceutical composition for preventing or treating cancer, including an interleukin-32θ mutant (IL-32θ mutant).

According to a preferred embodiment of the present invention, the interleukin-32θ mutant includes an amino acid sequence of SEQ ID NO: 1.

According to a preferred embodiment of the present invention, the interleukin-32θ mutant is encoded by a nucleotide sequence of SEQ ID NO: 2.

According to a preferred embodiment of the present invention, the interleukin-32θ mutant is encoded by a nucleotide sequence of SEQ ID NO: 3.

According to a preferred embodiment of the present invention, the cancer is at least any one selected from the group consisting of stomach cancer, lung cancer, liver cancer, pancreatic cancer, colon cancer, prostate cancer and breast cancer.

In addition, the present invention provides a pharmaceutical composition for preventing or treating an inflammatory disease, including an interleukin-32θ mutant (IL-32θ mutant).

According to a preferred embodiment of the present invention, the inflammatory disease is at least any one selected from the group consisting of arthritis, rheumatoid arthritis, gout, hepatitis, asthma, keratitis, gastritis, enteritis, nephritis, tuberculosis, bronchitis, pleurisy, peritonitis, spondylitis, pancreatitis, inflammatory pain, urethritis, cystitis, arteriosclerosis, sepsis, dermatitis, periodontitis and gingivitis.

In addition, the present invention provides an anti-cancer immunotherapeutic composition, including an interleukin-32θ mutant (IL-32θ mutant).

In addition, the present invention provides an anti-inflammatory immunotherapeutic composition, including an interleukin-32θ mutant (IL-32θ mutant).

In addition, the present invention provides a recombinant vector for expressing an interleukin-32θ mutant (IL-32θ mutant), including a nucleotide sequence of SEQ ID NO: 3.

In addition, the present invention provides the use of an interleukin-32θ mutant (IL-32θ mutant) for use in the treatment of cancer.

In addition, the present invention provides the use of an interleukin-32θ mutant (IL-32θ mutant) for use in the treatment of an inflammatory disease.

In addition, the present invention provides a method for treating cancer, including administering a composition including an interleukin-32θ mutant (IL-32θ mutant) to a cancer patient.

In addition, the present invention provides a method for treating an inflammatory disease, including administering a composition including an interleukin-32θ mutant (IL-32θ mutant) to a patient with an inflammatory disease.

### 1. Anti-cancer effect

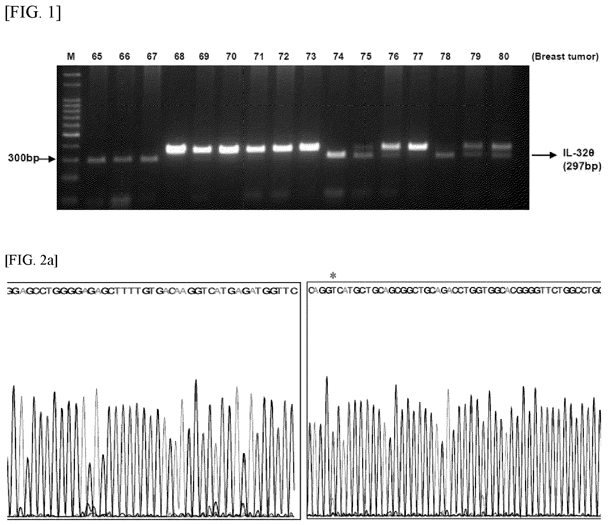

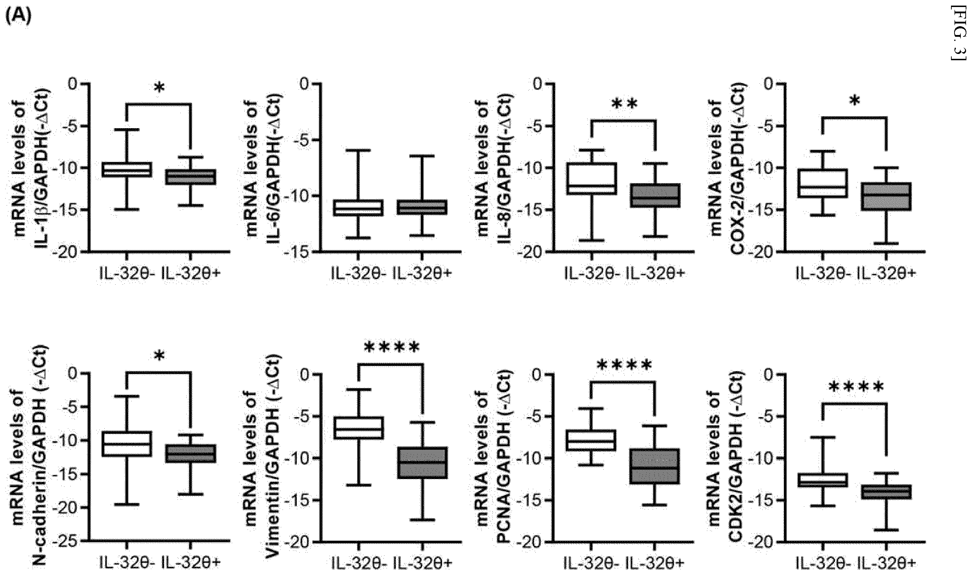

In the present invention, the inventors of the present invention discovered a 281^{st} point mutation (C to T) (IL-32θ(281T)) in tissues of breast cancer patients (n = 34) by using Sanger sequencing. Due to the limitations of Sanger sequencing, the inventors of the present invention used 34 IL-32θ RT-PCR products from selected patient tissue based on the intensity of PCR bands for sequencing. All selected RT-PCR product sequences harbored a C to T point mutation at position 281, which modifies alanine to valine at a 94^{th} codon (281C>T, Ala94Val; FIG. 2a). Additionally, the inventors of the present invention reordered RT-qPCR results from tumor tissue to investigate the effect of IL-32θ(281T) on tumor inflammation and breast cancer progression. In particular, inflammatory factors (IL-1β, IL-8, COX-2), mesenchymal markers (vimentin, N-cadherin) and proliferation-related factors (PCNA, CDK2) were significantly suppressed in tumor tissues expressing IL-32θ(281T). These results suggest that IL-32θ(281T) inhibits tumor progression and development (FIG. 3).

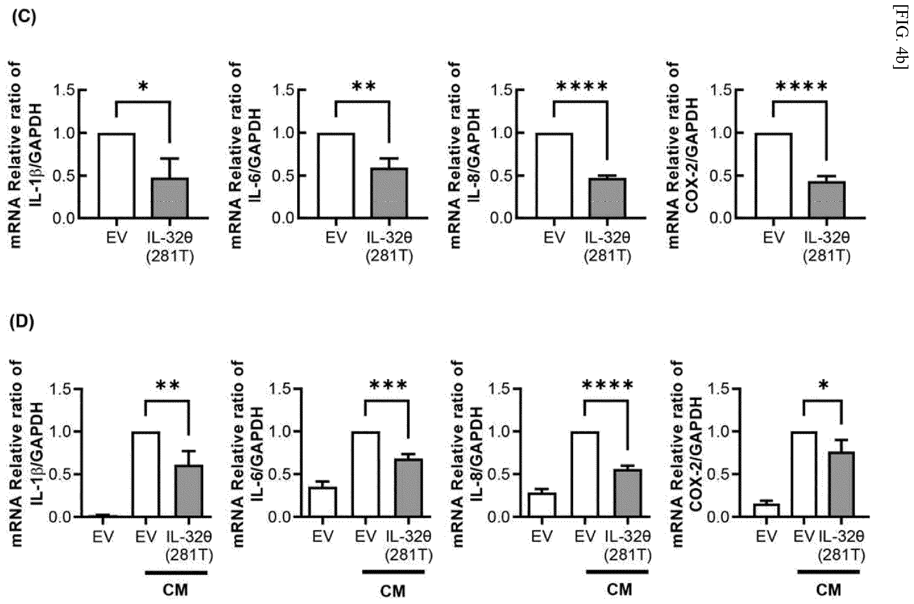

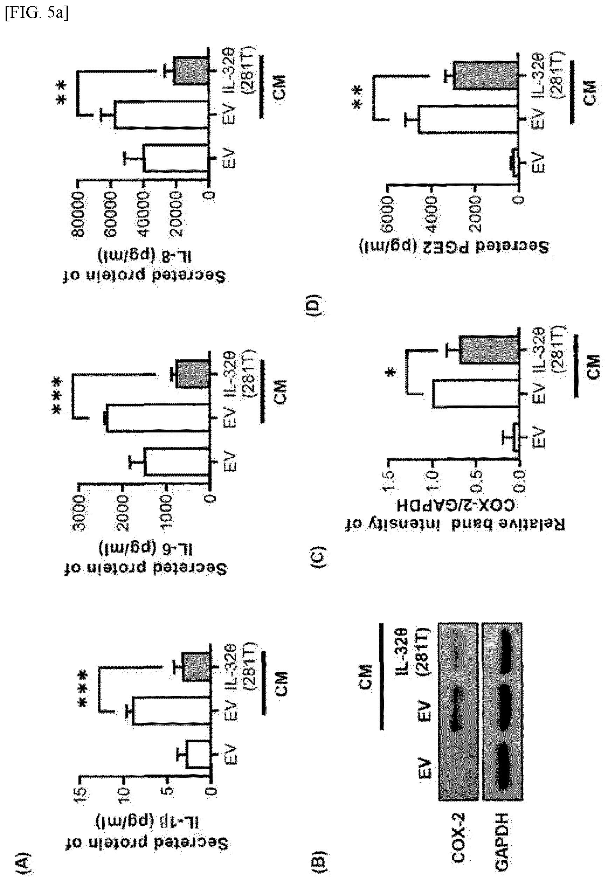

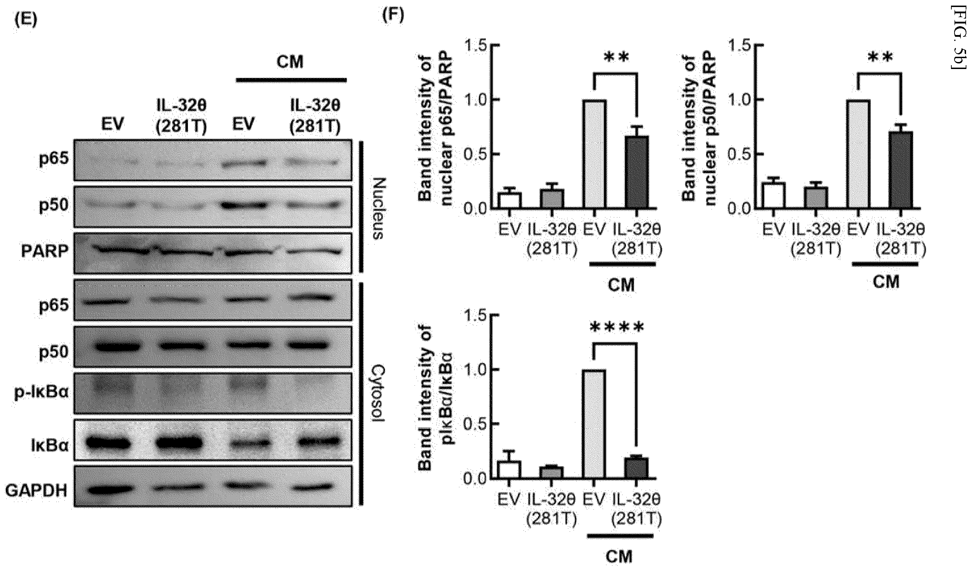

Inflammation is an immune system response to harmful stimuli, including bacterial and viral infections. However, many diseases are caused by inflammation. Various inflammatory factors have been found to promote tumor formation. IL-1β is a major cytokine of the IL-1 family that initiates tumor proliferation, angiogenesis and migration in breast cancer. IL-6 is a functional cytokine involved in inflammatory responses, angiogenesis, inhibition of apoptosis, cancer stem cell formation and migration of breast cancer. Likewise, IL-8 is associated with angiogenesis, tumor growth, proliferation and metastasis. COX-2 induces the conversion of arachidonic acid into prostaglandin H2 (PGH2), which is a precursor of prostaglandin E2 (PGE2). PGE2 is involved in tumorigenesis, tumor progression, angiogenesis and migration of breast cancer. The transcription factor NF-κB has been reported to induce inflammatory factors such as IL-1β, IL-6, IL-8 and COX-2. IκBα is an NF-κB binding protein that regulates NF-κB translocation through protein-protein interactions. The nuclear translocation of NF-κB is triggered by degradation of IκBα through AKT phosphorylation. Based on clinical results, MDA-MB-231, which is a highly aggressive triple-negative breast cancer cell line, was selected. RT-qPCR analysis showed that the mRNA levels of inflammatory factors were suppressed by IL-32θ(281T) in MDA-MB-231 cells (FIG. 4b). In addition, the protein expression level of COX-2 was determined by Western blotting, and the expression levels of secreted proteins such as IL-1β, IL-6, IL-8, and PGE2 were determined by using each ELISA kit (A, B, C of FIG. 5a). These results demonstrated that IL-32θ(281T) reduced the levels of inflammatory factors and PGE2 in CM-stimulated MDA-MB-231 cells compared to EV transfected cells (D of FIG. 5a). Additionally, IL-328(281T) inhibited the translocation of NF-κB into the nucleus of MDA-MB-231 cells stimulated with CM (FIG. 5b). These data suggest that inflammatory factors associated with tumor progression and aggressiveness are suppressed by IL-32θ(281T) in breast cancer cells.

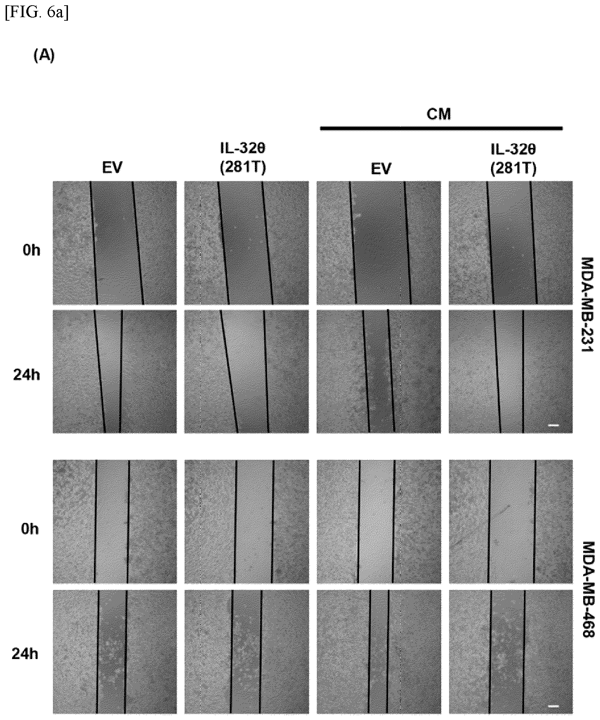

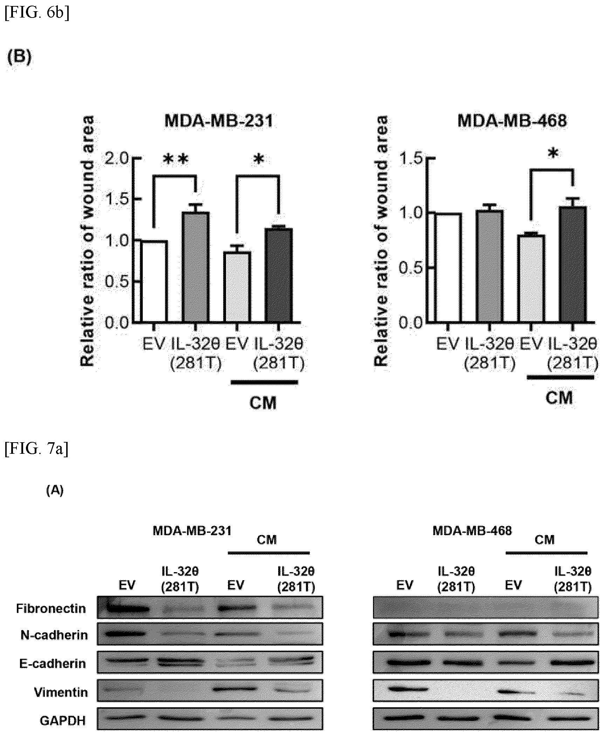

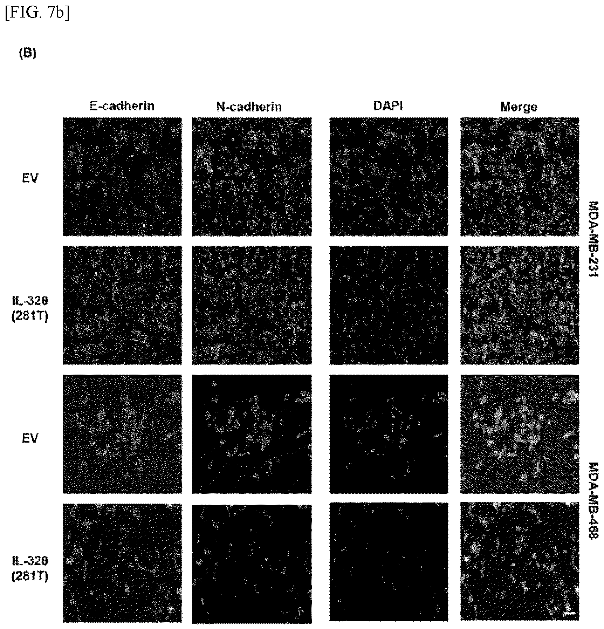

EMT is a transition step regulated and determined by various proteins. Mesenchymal cells express high levels of vimentin and fibronectin, which are associated with the cytoskeleton. Additionally, intercellular adhesion mediators (E-cadherin, N-cadherin) are essential proteins in determining epithelial and mesenchymal types. These factors are regulated by inflammatory factors through several signaling pathways. The inventors of the present invention investigated whether IL-32θ(281T) can regulate the migration of breast cancer cells. Compared to EV transfected cells, migration speed was significantly reduced in IL-32θ(281T)-expressing breast cancer cells (FIGS. 6a, 6b). Additionally, whereas Western blot analysis showed that mesenchymal markers (N-cadherin, vimentin, fibronectin) were downregulated by IL-32θ(281T) (FIGS. 7a, 7b), an epithelial marker (E-cadherin) was upregulated by IL-32θ(281T). Additionally, IF analysis demonstrated that whereas E-cadherin was increased by IL-32θ(281T) on the cell surface, N-cadherin was decreased (FIGS. 7a, 7b). These results support the fact that IL-32θ(281T) inhibits mesenchymal transition of breast cancer cells.

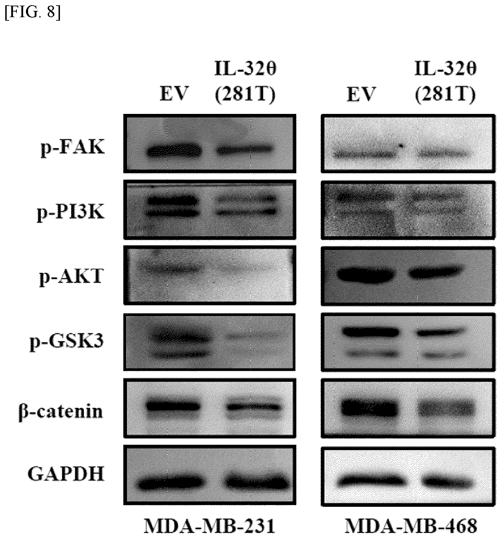

The nuclear translocation of β-catenin induces the transcription of mesenchymal and inflammatory factors in cancer. β-catenin degradation and translocation are regulated by interaction with glycogen synthase kinase 3β (GSK3β). The FAK/PI3K/AKT pathway induces the degradation of GSK3β through phosphorylation. Additionally, it has been reported that the FAK pathway is regulated by IL-32 through protein-protein interactions. In the present invention, Western blot analysis showed that FAK expression and phosphorylation were reduced by IL-32θ(281T). Additionally, the phosphorylation of PI3K and AKT was inhibited by IL-32θ(281T) through FAK inhibition. Finally, the degradation of β-catenin was increased by IL-32θ(281T) through inhibition of GSK3β degradation (FIG. 8).

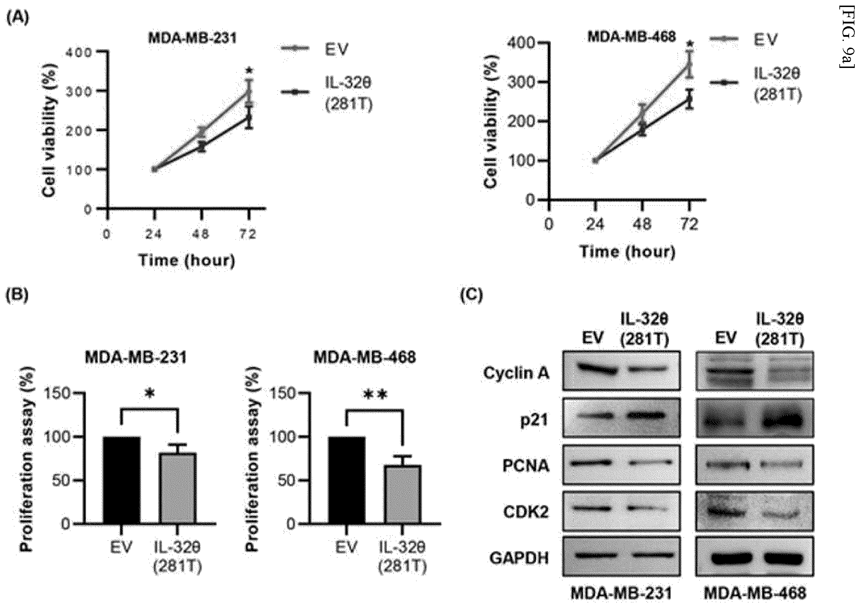

AKT is a well-known regulator of cell proliferation and survival in cancer. CDK and cyclin complexes induce proliferation through the regulation of cell cycle checkpoints. In particular, downregulation of the cyclin A/CDK2 complex induces S phase and G2/M phase cell cycle arrest. PCNA is a DNA clamp that supports DNA replication. Cyclin-dependent kinase inhibitor 1 (p21) binds to the CDK/cyclin complex and inhibits the cell cycle. The inventors of the present invention investigated whether IL-32θ(281T) can regulate the proliferation of breast cancer cells. IL-32θ(281T)-expressing cells showed reduced cell proliferation than EV-transfected cells (FIG. 9a). In addition, Western blotting analysis revealed that cell cycle-related factors (PCNA, cyclin A, cdk2, p21) are regulated by IL-32θ(281T) through the FAK/PI3K/AKT pathway (C in FIG. 9a). Flow cytometry analysis also showed that S phase and G2/M phase cell cycle arrest was induced by IL-32θ(281T) in breast cancer cells. These data support the idea that IL-32θ(281T) inhibits breast cancer proliferation by suppressing cell cycle-related factors (PCNA, cyclin A, cdk2, and p21) in breast cancer cells through the FAK/PI3K/AKT pathway.

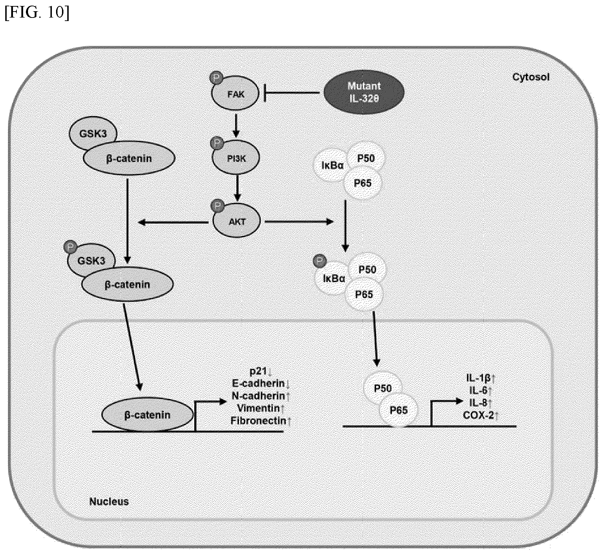

In conclusion, the inventors of the present invention identified a new mutation in IL-32θ that inhibits inflammation, EMT and breast cancer cell cycle by regulating intracellular NF-κB (p65/p50) and FAK/PI3K/AKT pathways (FIG. 10). IL-32θ(281T) of the present invention can be used in a potential therapeutic strategy to inhibit the progression of breast cancer due to its effects on inflammation, EMT and cell cycle arrest.

### 2. Anti-inflammatory effect

In the present invention, the inventors of the present invention investigated the cell surface receptor of IL-32θ (281T), in which cytosine is replaced with thymine at position 281 (281T) of IL-32θ, and evaluated its effect on human umbilical vein endothelial cells (HUVEC). Recombinant human IL-32θ(281T) was expressed, isolated and purified using Ni-NTA and IL-32 mAb(KU32-52)-coupled agarose column. The inventors of the present invention observed that IL-32θ(A94V) can bind to integrins αVβ3 and αVβ6, which suggests that integrins act as cell surface receptors for IL-32θ(A94V). In contrast to other isoforms such as IL-32β and IL-32γ, IL-32θ(A94V) significantly attenuated monocyte-endothelial adhesion by inhibiting the expression of intracellular adhesion molecule-1 (ICAM-1) and vascular adhesion molecule-1 (VCAM-1) in tumor necrosis factor (TNF)-α-stimulated HUVECs. IL-32θ(A94V) also reduced TNF-α-induced phosphorylation of protein kinase B (AKT) and c-jun N-terminal kinase (JNK) by inhibiting the phosphorylation of focal adhesion kinase (FAK). Additionally, IL-32θ(A94V) regulated the nuclear translocation of nuclear factor kappa B (NF-κB) and activator protein 1 (AP-1), which are involved in ICAM-1 and VCAM-1 expression. Monocyte-endothelial adhesion mediated by ICAM-1 and VCAM-1 is an important early step in atherosclerosis, which is a major cause of cardiovascular disease. The present invention suggests that IL-32θ(A94V) attenuates monocyte-endothelial adhesion by binding to the cell surface receptors integrins αVβ3 and αVβ6 and inhibiting the expression of ICAM-1 and VCAM-1 in HUVECs stimulated with TNF-α. These results demonstrate that IL-32θ(A94V) can act as an anti-inflammatory cytokine in chronic inflammatory diseases such as arteriosclerosis.

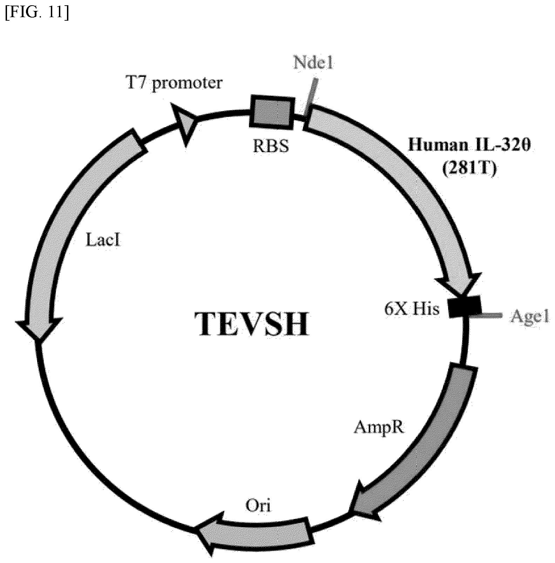

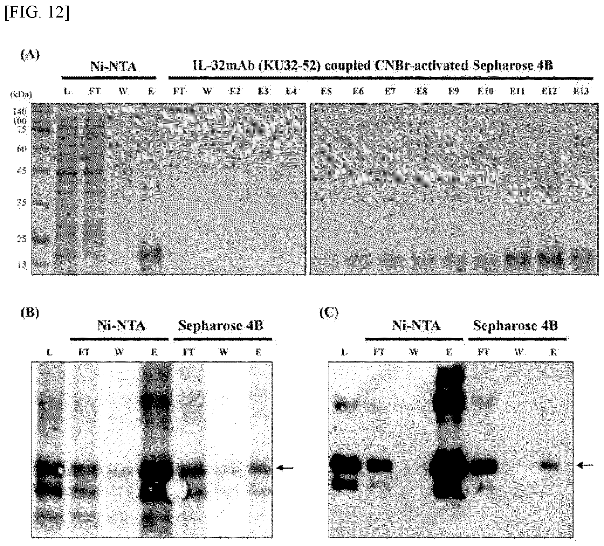

Specifically, the inventors of the present invention designed a recombinant vector using Nde1 and Age1 restriction enzymes (FIG. 11). The vector was transformed into Rosetta, which is one of the *E. coli* strains, using the heat shock method. The inserted DNA includes a His-tag for purification. Pure IL-32θ(A94V) was obtained using sequential purification of a Ni-NTA column and a CNBr-activated Sepharose 4B column coupled with IL-32 mAb KU32-52. The purified protein was confirmed by SDS-PAGE and Western blot (FIG. 12).

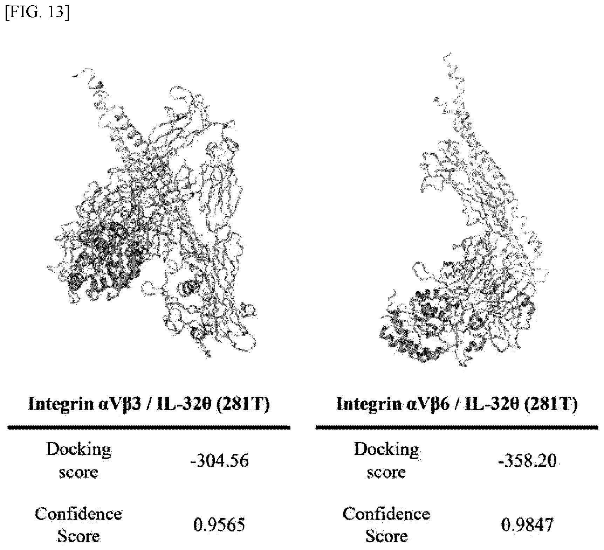

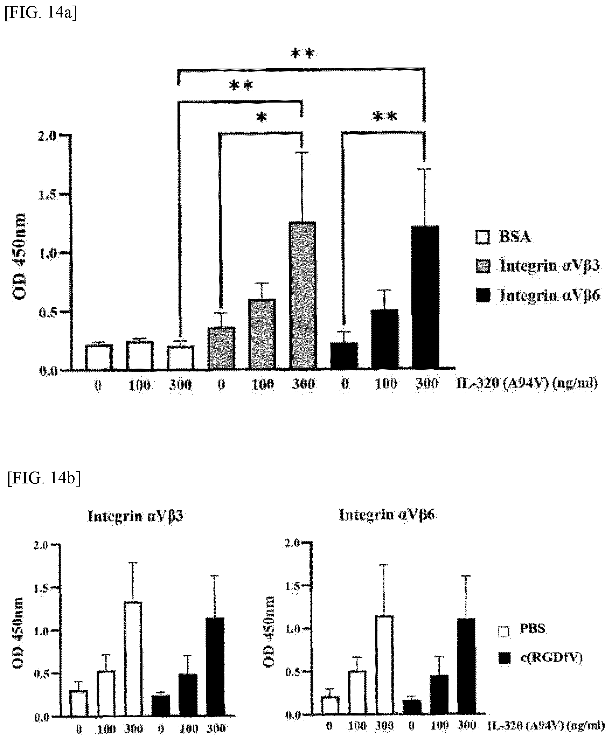

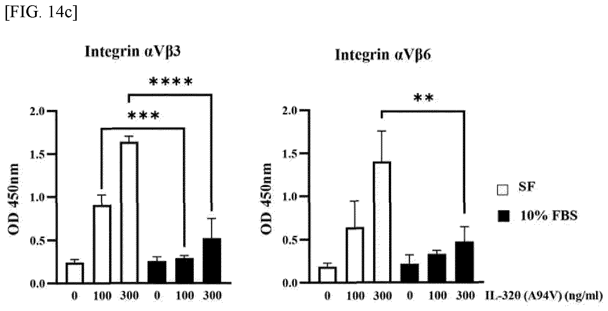

Most IL-32 isoforms, including IL-32θ(281T), have an RGD motif, and integrins αVβ3 and αVβ6 are known to bind to the RGD motif. Additionally, these integrins have been reported to bind IL-32α and IL-32β. Therefore, the inventors of the present invention hypothesized that IL-32θ(A94V) would also bind to these integrins. In protein-protein binding predictions, IL-32 showed a higher docking score with integrin αVβ6 than with integrin αVβ3 (FIG. 13). However, no significant differences were identified in the IL-32θ(A94V)-integrin binding analysis. IL-32θ(A94V) was found to bind both integrins αVβ3 and αVβ6 in a dose-dependent manner (FIG. 14a), suggesting that these integrins may act as receptors for IL-32θ(A94V). However, the interaction was not blocked by cyclo-(RGDfV) including the short RGD peptide, which indicates that, contrary to expectations, IL-32θ(A94V)-integrin binding is not mediated by the RGD motif (FIG. 14b). Extracellular matrices such as fibronectin, vitronectin and growth factors included in FBS are known to bind to integrins. They can bind to integrins and block the integrin-IL-32θ(A94V) interaction. As expected, the inventors of the present invention observed a decrease in interaction in medium containing 10% FBS compared to serum-free medium (FIG. 14c).

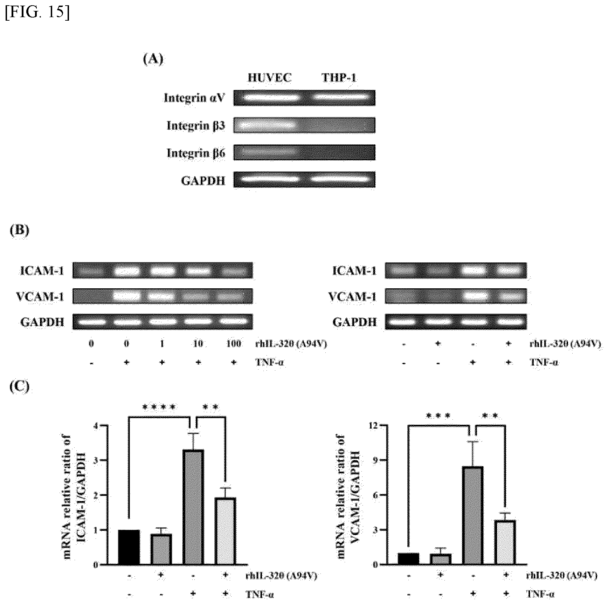

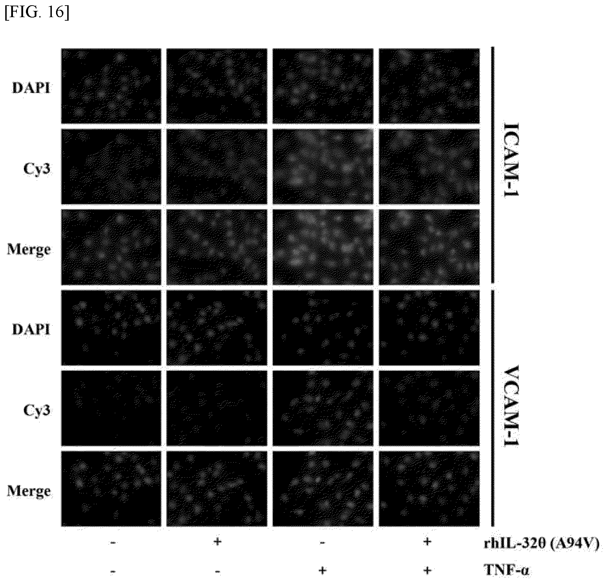

RT-PCR analysis confirmed the expressions of integrins αVβ3 and αVβ6 in HUVEC cells, but not in THP-1 human monocyte cells. The inventors of the present invention also demonstrated that the upregulation of the expressions of ICAM-1 and VCAM-1 induced by TNF-α was significantly reduced in IL-32θ(A94V)-pretreated HUVEC (FIGS. 15 and 16). These results show the opposite role of IL-32θ(A94V) compared to IL-32β and IL-32γ, which are known to induce the expression of these cell adhesion molecules.

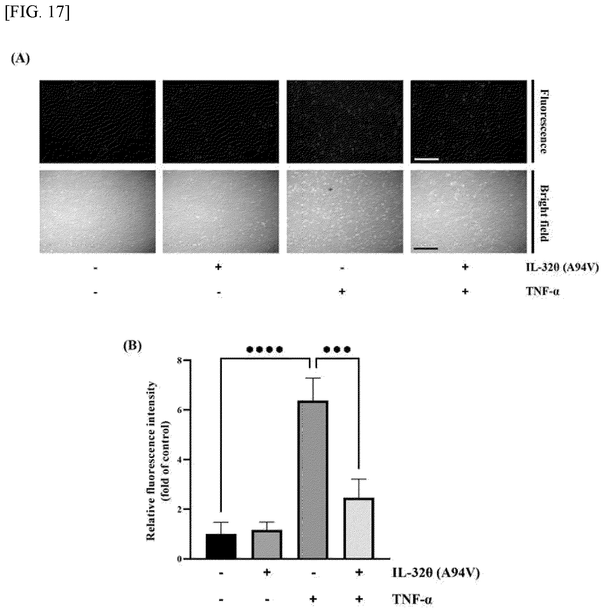

The inventors of the present invention investigated whether monocyte-endothelial adhesion mediated by these cell adhesion molecules is attenuated by IL-32θ(A94V). THP-1 monocytes were stained with the fluorescent dye calcein-AM and co-cultured with HUVEC. After several washes, the green staining fluorescence intensity of TNF-α treated THP-1 cells was significantly enhanced, whereas that of monocytes was decreased by IL-32θ(A94V) (FIG. 17). These results demonstrated that IL-32θ(A94V) attenuates monocyte endothelial adhesion by inhibiting the expressions of cell adhesion molecules ICAM-1 and VCAM-1 in HUVECs stimulated with TNF-α.

FAK is a well-known integrin-mediated signaling molecule that is closely involved in intracellular signaling induced by various cytokines and growth factors. Phosphorylated FAK induced by these molecules activates the AKT and JNK signaling pathways. The activation of AKT induces phosphorylation of IκB, which promotes nuclear translocation of NF-κB. JNK also causes the nuclear translocation of AP-1. Expression of ICAM-1 and VCAM-1 in HUVEC is accelerated by the transcription factors AP-1 and NF-κB. IL-32θ(A94V) downregulates this signaling pathway by inhibiting TNF-α-induced phosphorylation of FAK upstream of JNK and AKT, thereby attenuating ICAM-1 and VCAM-1 expression.

In summary, IL-32θ (A94V) of the present invention attenuated monocyte-endothelial adhesion by suppressing the expression of ICAM-1 and VCAM-1, which are key factors of atherosclerosis, through integrin-mediated signaling in HUVEC (FIG. 17). This evidence demonstrates a potential role for IL-32θ(A94V) in the treatment of chronic inflammatory diseases such as atherosclerosis.

Therefore, the present invention may provide a pharmaceutical composition for preventing or treating cancer, including an interleukin-32θ mutant (IL-32θ mutant).

According to a preferred embodiment of the present invention, the IL-32θ mutant may include part or all of an amino acid sequence of SEQ ID NO: 1.

The gene of the interleukin-32θ mutant of the present invention may have a partial nucleotide sequence of the existing interleukin-32θ deleted, and preferably, a partial nucleotide sequence of exon 6 is deleted, and more preferably, bases 144 to 203 of exon 6 may be deleted.

According to a preferred embodiment of the present invention, the IL-32θ mutant may be encoded by a nucleotide sequence of SEQ ID NO: 2.

According to a preferred embodiment of the present invention, the IL-32θ mutant may be encoded by a nucleotide sequence of SEQ ID NO: 3.

The nucleotide sequence of SEQ ID NO: 3 may be used to prepare or express a recombinant IL-32θ mutant protein more efficiently than the nucleotide sequence of SEQ ID NO: 2.

According to a preferred embodiment of the present invention, the cancer may be at least any one selected from the group consisting of stomach cancer, lung cancer, liver cancer, pancreatic cancer, colon cancer, prostate cancer and breast cancer.

As used herein, the term 'prevention' refers to all actions that suppress or delay the onset of diseases related to cancer or inflammatory diseases due to the interleukin-32θ mutant of the present invention.

As used herein, the term 'amelioration' or 'treatment' refers to all actions that ameliorate or benefit parameters related to cancer or inflammatory disease-related diseases, such as the degree of symptoms, due to the interleukin-32θ mutant of the present invention.

The pharmaceutical composition of the present invention may be in various parenteral dosage forms. When the composition is formulated, the composition may be prepared using one or more buffers (e.g., saline or PBS), antidiabetic agents, bacteriostats, chelating agents (e.g., EDTA or glutathione), fillers, extenders, binders, adjuvants (e.g., aluminum hydroxide), suspensions, thickeners, wetting agents, disintegrants or surfactants, diluents or excipients.

Formulations for parenteral administration include an aqueous sterile solution, a non-aqueous solvent, a suspension solvent, an emulsion, a freeze-dried preparation, a suppository or the like. As the non-aqueous solvent and the suspension solvent, it is possible to use propylene glycol, polyethylene glycol, a vegetable oil such as olive oil, an injectable ester such as ethyl oleate and the like. As a base of the suppository, it is possible to use Witepsol, Macrogol, Tween 61, cacao butter, laurin fat, glycerol, gelatin and the like.

The composition of the present invention may be administered orally or parenterally, and when administered parenterally, it may be formulated according to a method known in the art in the form of an injection for external use on the skin, intraperitoneal, rectal, intravenous, muscle, subcutaneous or intracerebrovascular injection.

The injection must be sterilized and protected from contamination by microorganisms such as bacteria and fungi. Examples of a suitable carrier for injection may be, but are not limited to, a solvent or a dispersion medium including water, ethanol, polyols (e.g., glycerol, propylene glycol, liquid polyethylene glycol, etc.), mixtures thereof and/or vegetable oils. More preferably, as a suitable carrier, it is possible to use an isotonic solution such as Hank's solution, Ringer's solution, triethanolamine-containing phosphate buffered saline (PBS) or sterile water for injection, 10% ethanol, 40% propylene glycol, and 5% dextrose and the like. In order to protect the injection from microbial contamination, various antimicrobial agents and antifungal agents such as paraben, chlorobutanol, phenol, sorbic acid, and thimerosal may be additionally included. Furthermore, in most cases, the injection may additionally include an isotonic agent such as sugar or sodium chloride.

The composition of the present invention is administered in a pharmaceutically effective amount. The pharmaceutically effective amount refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period, factors including simultaneously used drugs and other factors well known in the medical field. The composition of the present invention may be administered as an individual therapeutic agent or in combination with other therapeutic agents, may be administered sequentially or simultaneously with therapeutic agents in the related art, and may be administered in a single dose or multiple doses. That is, the total effective amount of the composition of the present invention may be administered to a patient in a single dose or may be administered by a fractionated treatment protocol, in which multiple doses are administered over a long period of time. It is important to administer the composition in a minimum amount that can obtain the maximum effect without any side effects, in consideration of all the aforementioned factors, and this amount may be easily determined by a person skilled in the art.

The composition of the present invention may be used alone or in combination with surgery, radiation therapy, hormone therapy, chemotherapy and methods using biological response modifiers.

In addition, the present invention may provide a pharmaceutical composition for preventing or treating an inflammatory disease, including the IL-32θ mutant.

Since the interleukin-32θ mutant and the pharmaceutical composition are the same as the concept used in the pharmaceutical composition for preventing or treating cancer, the explanation is replaced with the description thereof.

According to a preferred embodiment of the present invention, the inflammatory disease may be at least any one selected from the group consisting of arthritis, rheumatoid arthritis, gout, hepatitis, asthma, keratitis, gastritis, enteritis, nephritis, tuberculosis, bronchitis, pleurisy, peritonitis, spondylitis, pancreatitis, inflammatory pain, urethritis, cystitis, arteriosclerosis, sepsis, dermatitis, periodontitis and gingivitis.

In addition, the present invention may provide an anti-cancer immunotherapeutic composition or an anti-inflammatory immunotherapeutic composition, including an interleukin-32θ mutant (IL-32θ mutant).

The immunotherapeutic agent may refer to a therapeutic agent that treats a disease by promoting or suppressing the body's immune response while minimizing side effects by using the characteristics of the body's immune cells.

The interleukin-32θ mutant of the present invention is a new anti-inflammatory cytokine with a completely new function that is differentiated from other IL-32s, and it may be used as an immunotherapeutic agent to prevent and treat various cancers and inflammatory diseases. The cancer may be at least any one selected from the group consisting of stomach cancer, lung cancer, liver cancer, pancreatic cancer, colon cancer, prostate cancer and breast cancer.

The inflammatory disease may be at least any one selected from the group consisting of arthritis, rheumatoid arthritis, gout, hepatitis, asthma, keratitis, gastritis, enteritis, nephritis, tuberculosis, bronchitis, pleurisy, peritonitis, spondylitis, pancreatitis, inflammatory pain, urethritis, cystitis, arteriosclerosis, sepsis, dermatitis, periodontitis and gingivitis.

Since the interleukin-32θ mutant is the same as the concept used in the pharmaceutical composition for preventing or treating cancer, the explanation is replaced with the description thereof.

In addition, the present invention may provide a recombinant vector for expressing an interleukin-32θ mutant (IL-32θ mutant), including a nucleotide sequence of SEQ ID NO: 3.

The nucleotide sequence of SEQ ID NO: 3 may be used to prepare or express a recombinant IL-32θ mutant protein more efficiently than the nucleotide sequence of SEQ ID NO: 2.

Since the interleukin-32θ mutant is the same as the concept used in the pharmaceutical composition for preventing or treating cancer, the explanation is replaced with the description thereof.

In addition, the present invention may provide the use of an interleukin-32θ mutant (IL-32θ mutant) for use in the treatment of cancer.

Since the interleukin-32θ mutant is the same as the concept used in the pharmaceutical composition for preventing or treating cancer, the explanation is replaced with the description thereof.

According to a preferred embodiment of the present invention, the cancer may be at least any one selected from the group consisting of stomach cancer, lung cancer, liver cancer, pancreatic cancer, colon cancer, prostate cancer and breast cancer.

In addition, the present invention may provide the use of an interleukin-32θ mutant (IL-32θ mutant) for use in the treatment of an inflammatory disease.

Since the interleukin-32θ mutant is the same as the concept used in the pharmaceutical composition for preventing or treating cancer, the explanation is replaced with the description thereof.

According to a preferred embodiment of the present invention, the inflammatory disease may be at least any one selected from the group consisting of arthritis, rheumatoid arthritis, gout, hepatitis, asthma, keratitis, gastritis, enteritis, nephritis, tuberculosis, bronchitis, pleurisy, peritonitis, spondylitis, pancreatitis, inflammatory pain, urethritis, cystitis, arteriosclerosis, sepsis, dermatitis, periodontitis and gingivitis.

In addition, the present invention may provide a method for treating cancer, including administering a composition including an interleukin-32θ mutant (IL-32θ mutant) to a cancer patient.

Since the interleukin-32θ mutant is the same as the concept used in the pharmaceutical composition for preventing or treating cancer, the explanation is replaced with the description thereof.

According to a preferred embodiment of the present invention, the cancer may be at least any one selected from the group consisting of stomach cancer, lung cancer, liver cancer, pancreatic cancer, colon cancer, prostate cancer and breast cancer.

Since the administration is the same as the concept used in the pharmaceutical composition for preventing or treating cancer, the explanation is replaced with the description thereof.

In addition, the present invention may provide a method for treating an inflammatory disease, including administering a composition including an interleukin-32θ mutant (IL-32θ mutant) to a patient with an inflammatory disease.

Since the interleukin-32θ mutant is the same as the concept used in the pharmaceutical composition for preventing or treating cancer, the explanation is replaced with the description thereof.

According to a preferred embodiment of the present invention, the inflammatory disease may be at least any one selected from the group consisting of arthritis, rheumatoid arthritis, gout, hepatitis, asthma, keratitis, gastritis, enteritis, nephritis, tuberculosis, bronchitis, pleurisy, peritonitis, spondylitis, pancreatitis, inflammatory pain, urethritis, cystitis, arteriosclerosis, sepsis, dermatitis, periodontitis and gingivitis.

Since the administration is the same as the concept used in the pharmaceutical composition for preventing or treating cancer, the explanation is replaced with the description thereof.

### Advantageous Effects

The interleukin-32θ mutant of the present invention {IL-32θ mutant, IL-32θ(281T) or IL-32θ(A94V)} regulates the NF-κB pathway to suppress the expression of inflammatory factors and the FAK/PI3K/AKT pathway, and it is possible to control the progression of cancer, especially breast cancer, by suppressing EMT markers and cell cycle-related factors. In addition, IL-32θ(281T) binds to integrins αVβ3 and αVβ6, which are the cell surface receptors, and inhibits the expressions of ICAM-1 and VCAM-1 in HUVECs stimulated with TNF-α, thereby weakening monocyte-endothelial adhesion, and thus, it is possible to provide anti-inflammatory effects in chronic inflammatory diseases such as arteriosclerosis. In addition, it can be utilized as a research material for various cancers and/or inflammatory diseases.

### Description of Drawings

FIG. 1 shows some RT-PCR results for mRNA expressions of IL-32θ and IL-32β in breast cancer tissues (tissue numbers 65 to 80). Primers targeting the sequence from exon 5 to exon 7 (forward primer: 5'-ACGTGGACAGGTGATGTCGA-3, SEQ ID NO: 7'; reverse primer: 5'-GAGCAGCAGAAACTCTGGAA-3', SEQ ID NO: 8) were designed to detect IL-32θ (297 bp).
FIG. 2a shows one of the Sanger sequencing data from breast cancer and surrounding normal tissue. Point mutations in IL-32θ were found in 34 randomly selected samples of breast tumor tissue (n = 16) and adjacent normal tissue (n = 18). IL-32θ mRNA was amplified by RT-PCR, and DNA was isolated using a gel extraction kit.

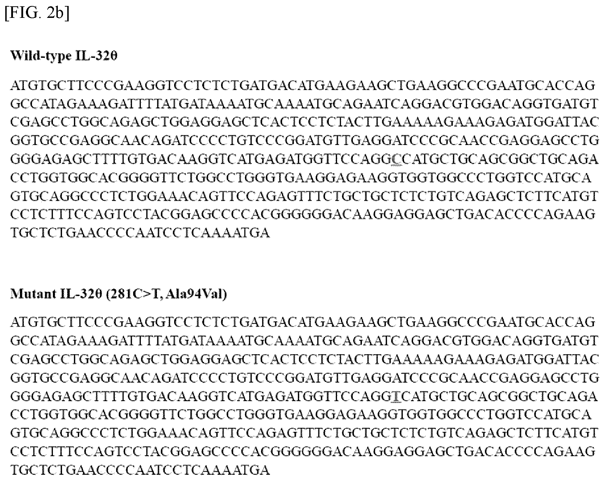

FIG. 2b shows the sequences of wild-type IL-32θ (SEQ ID NO:4) and mutant IL-32θ(281T) (SEQ ID NO:2). Mutation points are highlighted in red. The wild-type sequence was retrieved from GenBank (FJ985780.1).
FIG. 3 shows RT-qPCR results of inflammatory factors (IL-1β, IL-6, IL-8, COX-2), EMT factors (N-cadherin, vimentin) and proliferation factors (CDK2, PCNA). The line across the box represents the median. Statistical significance was analyzed using Student's t-test (*p < 0.05, **p < 0.01, ***p < 0.001, ****p < 501 0.0001, n = 90).

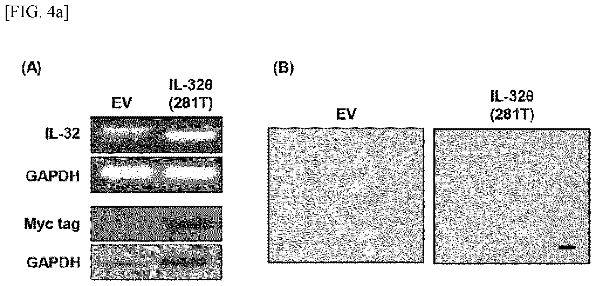

FIG. 4a shows the modulatory effect of IL-32θ(281T) on the mRNA levels of inflammatory factors in MDA-MB-231 cells. (A) Lipofectamine was used to establish EV- and IL-32θ(281T)-overexpressing MDA-MB-231 cells. (B) shows cell morphological changes in transfected MDA-MB-231 cells. Scale bar, 50 µm.
FIG. 4b shows the modulatory effect of IL-32θ(281T) on the mRNA levels of inflammatory factors in MDA-MB-231 cells. (C, D) The mRNA levels of inflammatory factors were confirmed by RT-qPCR with or without tumor-associated macrophage-like M2 macrophage secretion conditioned medium (CM). Results represent the mean ± SD of three experiments (One-way ANOVA with Student's t-test and Tukey's honest test *p < 0.05, **p < 0.01, ***p < 0.001, ****p < 0.0001, n = 3).
FIG. 5a shows the modulatory effect of IL-32θ(281T) on cytokine expression and nuclear translocation of NF-κB. (A) Secretion levels of IL-1β, IL-6 and IL-8 were analyzed by ELISA using supernatants obtained from MDA-MB-231 cells stimulated with CM. (B, C) Protein levels of COX-2 were analyzed by Western blotting in MDA-MB-231 cells stimulated with CM. Intensity was quantified using ImageJ. (D) PGE2 levels were quantified by competition ELISA. Bar graphs show the intensity of Western blotting bands after normalization to the internal control for each sample. Results represent the mean ± standard deviation of three experiments (One-way ANOVA with Tukey's honest test *p < 0.05, **p < 0.01, ***p < 0.001, ****p < 0.0001, n = 3).
FIG. 5b shows the modulatory effect of IL-32θ(281T) on cytokine expression and nuclear translocation of NF-κB. (E, F) show the modulatory effect of IL-32θ(281T) on the nuclear translocation of NF-κB obtained by Western blot analysis. Bar graphs show the intensity of Western blotting bands after normalization to the internal control for each sample. Intensity was quantified using Image J. Results represent the mean ± standard deviation of three experiments (One-way ANOVA with Tukey's honest test *p < 0.05, **p < 0.01, ***p < 0.001, ****p < 0.0001, n = 3).
FIG. 6a shows the modulatory effect of IL-32θ(281T) on breast cancer cell migration. For wound healing analysis, MDA-MB-231 cells (1.5 × 10⁵ cells/well) and MDA-MB-468 cells (3 × 10⁵ cells/well) were seeded in 24-well plates. Plates were scraped using a sterile 200 µL pipette tip and washed with PBS, and serum-free medium with or without CM was added for 24 hours. Scale bar, 200 µm. Wound area was quantified using Image J.
FIG. 6b shows the modulatory effect of IL-32θ(281T) on breast cancer cell migration. For wound healing analysis, MDA-MB-231 cells (1.5 × 10⁵ cells/well) and MDA-MB-468 cells (3 × 10⁵ cells/well) were seeded in 24-well plates. Plates were scraped using a sterile 200 µL pipette tip and washed with PBS, and serum-free medium with or without CM was added for 24 hours. Wound area was quantified using Image J. Results represent the mean ± standard deviation of three experiments (One-way ANOVA with Tukey's honest test *p < 0.05, **p < 0.01, ***p < 0.001, ****p < 0.0001, n= 3).
FIG. 7a shows the modulatory effect of IL-32θ(281T) on epithelial-mesenchymal transition markers of breast cancer cells. The expression of epithelial-mesenchymal transition-related proteins (fibronectin, N-cadherin, vimentin, E-cadherin) was analyzed by Western blotting.
FIG. 7b shows the modulatory effect of IL-32θ(281T) on epithelial-mesenchymal transition markers in breast cancer cells. E-cadherin and N-cadherin expression was analyzed by immunofluorescence.
FIG. 8 shows the results of Western blotting analysis of the modulatory effect of IL-32θ (281T) on signaling pathways (FAK, PI3K, AKT, GSK3β, β-catenin) in breast cancer cells. EV- and IL-32θ-transfected breast cancer cells (2 × 10⁵ cells/mL) were seeded in 60pi dishes and cultured for 48 hours.
FIG. 9a shows the modulatory effect of IL-32θ(281T) on the proliferation of breast cancer cells. (A) shows the results of time-dependent cell viability analysis of EV- and IL-32θ(281T)-transfected breast cancer cells. (B) shows the results of cell proliferation analysis of EV- and IL-32θ(281T)-transfected breast cancer cells. EV- and IL-32θ(281T)-transfected breast cancer cells were cultured for 72 hours. (C) shows the effect of IL-32θ(281T) on the expression of cell cycle-related factors (cyclin A, Cdk2, PCNA, p21) in breast cancer cells measured by Western blotting.

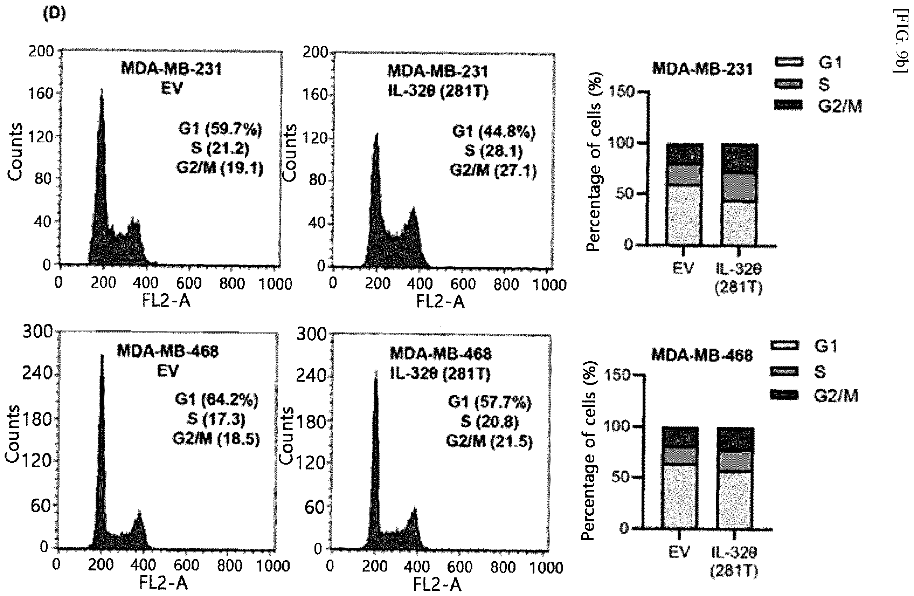

FIG. 9b shows the modulatory effect of IL-32θ(281T) on the proliferation of breast cancer cells. (D) shows cell cycle arrest analyzed by PI staining and flow cytometry. Results represent the mean ± SD of three experiments (Student's t-test *p < 0.05, **p < 0.01, ***p < 0.001, ****p < 0.0001, n = 3).
FIG. 10 shows a schematic diagram of the modulatory effect of mutated IL-32θ(281T) on intracellular pathways. IL-32θ(281T) inhibits the phosphorylation of FAK and IκBα. IL-32θ(281T) inhibits the expression and translocation of β-catenin by inhibiting phosphorylated FAK. Additionally, NF-κB is inhibited by IL-32θ(281T) through the inhibition of phosphorylated IκBα. Therefore, IL-32θ(281T) reduces breast cancer migration, proliferation and inflammation through FAK-PI3K-GSK3 and NF-κB pathways.
FIG. 11 shows a schematic diagram of mutant IL-32θ(281T) expression vector and IL-32θ(281T) purification. TEVSH with pTH24 as the backbone was used as the protein expression vector. A His tag was included for purification. Recombinant DNA was constructed using Nde1 and Age1 restriction enzymes.
FIG. 12 shows the results of expression and purification of IL-32θ(281T) using SDS-PAGE (A) and Western blot using KU32-52 mAb (B) or anti-His tag mAb (C). Mutant IL-32θ(281T) was purified twice on a Ni-NTA column followed by IL-32mAb (KU32-52)-coupled CNBr-activated Sepharose 4B. IL-32θ (281T) bound to the Ni-NTA column was eluted with 500 mM imidazole, and additional purification was performed by IL-32 mAb (KU32-52)-coupled CNBr-activated Sepharose 4B using low pH Tris-glycine buffer (pH 3.0) (L, lysate of *E. coli* including the IL-32θ(281T) expression vector; FT, flow through; W, washing flow; E, elution; E2 to E13, eluted using low pH Tris-glycine buffer (pH 3.0)).
FIG. 13 shows the 3D structural modeling results of IL-32θ(A94V) and IL-32θ(A94V)-integrin binding prediction. Protein-protein binding was predicted by the HDOCK server (IL-32θ(A94V): yellow, integrin: orange). The tertiary structure of IL-32θ(A94V) was constructed by I-TASSER, and the integrin was provided by PDB.
FIG. 14a shows IL-32θ(A94V) binding to recombinant αVβ3 and αVβ6 integrins. IL-32θ(A94V) binds to recombinant αVβ3 and αVβ6 integrins. Binding of IL-32θ (A94V) to integrins αVβ3 and αVβ6 was assessed using IL-32 mAb (KU32-52). Results represent the mean ± standard deviation of three experiments (*p < 0.05, **p < 0.01, ***p < 0.001, ****p < 0.0001 by one-way ANOVA).
FIG. 14b shows IL-32θ(A94V) binding to recombinant αVβ3 and αVβ6 integrins. The effect of cyclo-(RGDfV) on the binding of IL-32θ(A94V) to recombinant αVβ3 and αVβ6 integrins is shown. Binding of IL-32θ (A94V) to integrins αVβ3 and αVβ6 was assessed using IL-32 mAb (KU32-52). Results represent the mean ± standard deviation of three experiments (*p < 0.05, **p < 0.01, ***p < 0.001, ****p < 0.0001 by one-way ANOVA).
FIG. 14c shows IL-32θ(A94V) binding to recombinant αVβ3 and αVβ6 integrins. The effect of FBS on the binding of IL-32θ(A94V) to recombinant αVβ3 and αVβ6 integrins is shown. Binding of IL-32θ (A94V) to integrins αVβ3 and αVβ6 was assessed using IL-32 mAb (KU32-52). Results represent the mean ± standard deviation of three experiments (*p < 0.05, **p < 0.01, ***p < 0.001, ****p < 0.0001 by one-way ANOVA).
FIG. 15 shows the effect of IL-32θ(A94V) on mRNA expression of ICAM-1 and VCAM-1 in TNF-α stimulated human umbilical vein endothelial cells (HUVEC). The expression of integrin αV, β3 and β6 subunits was confirmed by RT-PCR in HUVEC cells but not in THP-1 human monocyte cells (A). HUVECs were pretreated with IL-32θ(A94V) (100 ng/mL) for 1 hour and stimulated with TNF-α (10 ng/mL) for 4 hours. mRNA expression of adhesion molecules was detected by RT-PCR (A) and RT-qPCR (B) analysis. Results represent the mean ± SD of three experiments (** p < 0.01, *** p < 0.001, **** p < 0.0001 by one-way ANOVA).
FIG. 16 shows the effect of IL-32θ(A94V) on protein expression of ICAM-1 and VCAM-1 in TNF-α stimulated human umbilical vein endothelial cells (HUVEC). HUVECs were pretreated with IL-32θ(A94V) (100 ng/mL) for 1 hour and stimulated with TNF-α (10 ng/mL) for 6 hours. Protein expression of adhesion molecules was analyzed by immunofluorescence using specific antibodies.
FIG. 17 IL-32θ(A94V) attenuates monocyte adhesion to HUVEC. (A) shows a fluorescent picture of calcein 665 AM-labeled THP-1 cells (green) adhesion to HUVEC. HUVECs were pretreated with IL-32θ(A94V) (100 ng/ml) for 1 hour and then stimulated with TNF-α for 6 hours. Then, HUVECs were incubated with calcein-AM-labeled THP-1 for 30 minutes. (B) Quantified THP-1-HUVEC adhesion results were normalized to the control group. Fluorescence was measured using Image J software. The scale bar in each image represents 650 µm. Results represent the mean ± standard deviation of three experiments (**** p < 0.0001 by one-way ANOVA).

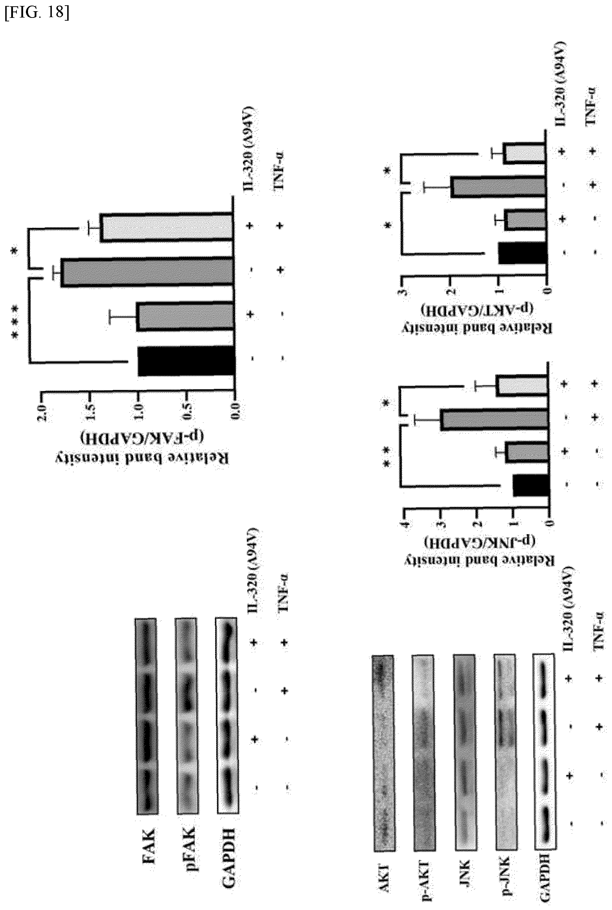

FIG. 18 shows the effect of IL-32θ(A94V) on the phosphorylation levels of FAK, JNK and AKT in HUVEC cells. HUVECs were pretreated with IL-32θ(A94V) (100 ng/mL) for 1 hour and then stimulated with TNF-α for 10 minutes (FAK) or 15 minutes (JNK, AKT). Phosphorylation levels of FAK, AKT and JNK were confirmed by Western blot, and band intensities were quantified using Image J software. Results represent the mean ± SD of three experiments (*p < 0.05, **p < 0.01, ***p < 0.001 by one-way ANOVA).

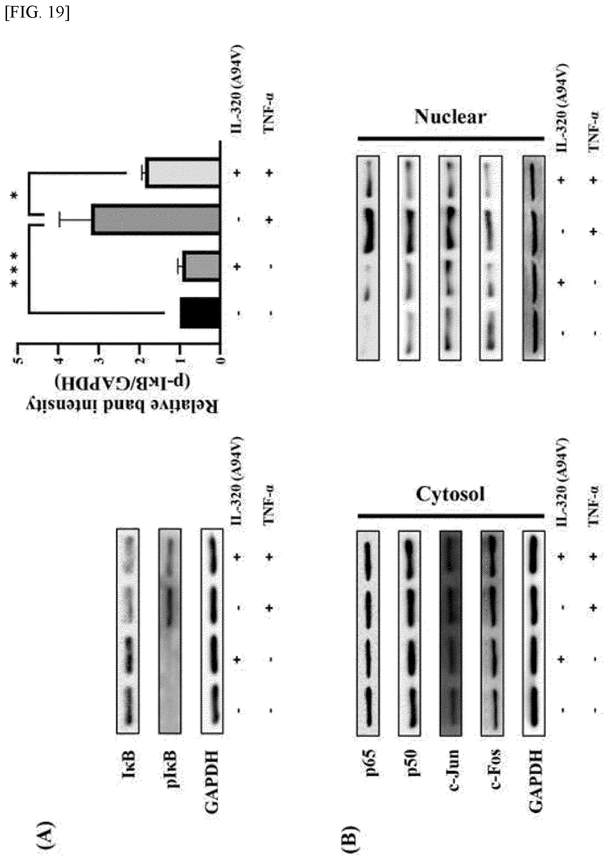

FIG. 19 shows the effect of IL-32θ (A94V) on the phosphorylation level of IκB and nuclear translocation of NF-κB (p65/p50) and AP-1 (c-Fos/c-Jun) in HUVEC cells. HUVEC cells were incubated with IL-32θ (A94V) for 1 hour and then stimulated with TNF-α (10 ng/ml) for 10 minutes (IκB) or 30 minutes (transcription factor). Harvested cells were subjected to nuclear fractionation. Phosphorylation and translocation levels were analyzed by Western blot, and band intensities were quantified using ImageJ software. Results represent the mean ± SD of three experiments (*p < 0.05, ***p < 0.001 by one-way ANOVA).

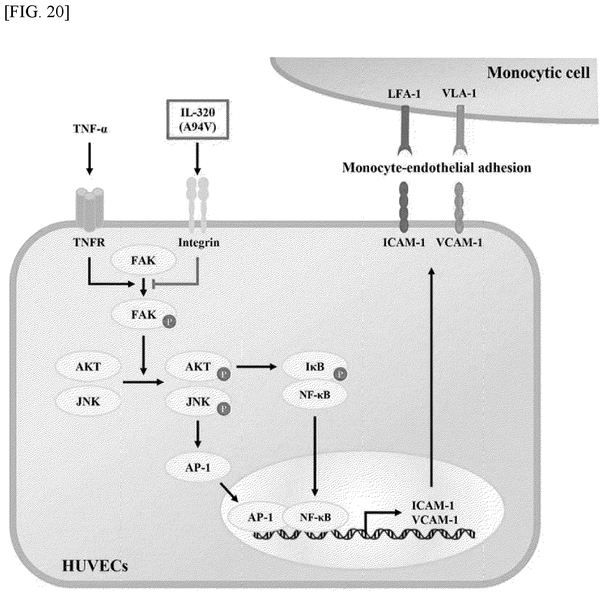

FIG. 20 shows a schematic diagram of signaling pathways involved in TNF-α induced ICAM-1 and VCAM-1 expression and integrin-IL-32θ(A94V) binding effect. TNF-α-induced phosphorylation of FAK, AKT and JNK and nuclear translocation of NF-κB and AP-1 promote the expression of ICAM-1 and VCAM-1. IL-32θ(A94V) inhibits the expression of ICAM-1 and VCAM-1 by inhibiting these intracellular signaling pathways.

### Modes of the Invention

### 1. Anti-cancer effect

### [Example 1]

### Patient samples

The biospecimens of the present invention included breast tumors (n = 90) and adjacent normal tissue (n = 90). Biological samples from breast cancer patients used in the present invention were provided by Chonnam National University Hwasun Hospital Biobank (Hwasun-gun, Korea) and Korea University Guro Hospital (Seoul, Korea). This study was approved by the Konkuk University Institutional Review Board (7001355-201704-E-047), and all subjects gave informed consent. mRNA was extracted from frozen tissue using a homogenizer and TRI Reagent^{®} (Ambion, Austin, TX, USA), and cDNA was synthesized using M-MuLV reverse transcriptase (New England Biolabs, Beverly, MA, USA) according to the manufacturer's instructions.

### [Example 2]

### Discovery of mutated IL-32θ in breast tissue

The mRNA expression of IL-32θ in tissues of breast cancer patients was confirmed using specific primer sets based on size differences (FIG. 1). The inventors of the present invention used a primer set that can distinguish isotypes depending on the size of the PCR product. Sequencing of IL-32θ was performed in samples strongly expressing a gene (n = 34) at Bionics (Seoul, Korea) using the Sanger method with an ABI 3730XL (Thermo Fisher Scientific, Waltham, MA, USA) and a BigDye Terminator v3.1 cycle sequencing kit (Thermo Fisher Scientific). A point mutation was detected in the IL-32θ sequence (FIG. 2a). The point mutation was located between exons 7 and 8 of IL-32θ, outside the splice site. The point mutation site was the 94^{th} codon, counting from the start codon (FIG. 2b). Additionally, sequencing data from 34 randomly selected samples of IL-32θ showed that the 94^{th} amino acid was mutated from alanine to valine. Therefore, the inventors of the present invention named this mutation IL-32θ(281T) or IL-32θ(A94V) (n = 34).

### [Example 3]

### mRNA expression of inflammatory, migration and proliferation factors in tumor tissue

Inflammatory factors (IL-1β, IL-6, IL-8, COX-2), mesenchymal markers (vimentin, N-cadherin) and proliferation factors (the mRNA levels of PCNA, CDK2) were measured. Breast tumor tissues were divided into two groups according to IL-32θ(281T) expression obtained by RT-PCR (Table 1).

**[Table 1]**

| Whether IL-32θ is expressed | Breast tumor tissue (n=90) | Normal tissue (n=90) |
|---|---|---|
| IL-32θ- | 50 | 40 |
| IL-32θ+ | 40 | 50 |

Specifically, RT-qPCR was performed with a relative quantification protocol using the BioFact^{™}2X Real-time PCR Kit (BioFact, Daejeon, Korea) and Rotor-Gene 6000 series software 1.7 (Qiagen, Venlo, Netherlands). Primer sequences are listed in [Table 2]. Transcript levels were calculated using the ^{△△}Ct or ^{△}Ct method.

**[Table 2]**

| Gene | Direction | Primer Sequence (5'→3') | SEQ ID NO: |
|---|---|---|---|
| GAPDH | Forward direction | AGAACATCATCCCTGCCTCT | 5 |
| | Reverse direction | CTGCTTCACCACCTTCTTGA | 6 |
| IL-32θ | Forward direction | ACGTGGACAGGTGATGTCGA | 7 |
| | Reverse direction | GAGCAGCAGAAACTCTGGAA | 8 |
| IL-1β | Forward direction | CGACAGACCTTCCAGGAGAATC | 9 |
| | Reverse direction | GTGCAGTTCAGT GATCGTACACC | 10 |
| IL-6 | Forward direction | AGACAGCCACTCACCTCTTCAG | 11 |
| | Reverse direction | TTCTGCCAGTGCCTCTTTGCTG | 12 |
| IL-8 | Forward direction | GGTCTGTGTGAAGGTGCAGT | 13 |
| | Reverse direction | GTTTTCCTTGGGGTCCAGAC | 14 |
| COX-2 | Forward direction | CGGTGAAACTCTGGCTAGACAG | 15 |
| | Reverse direction | GCAAACCGTAGATGCTCAGGGA | 16 |
| N-cadherin | Forward direction | CCT CCA GAG TTT ACT GCC ATG AC | 17 |
| | Reverse direction | GTA GGA TCT CCG CCA CTG ATT C | 18 |
| Vimentin | Forward direction | AGGCAAAGCAGGAGTCCACTGA | 19 |
| | Reverse direction | ATCTGGCGTTCCAGGGACTCAT | 20 |
| PCNA | Forward direction | CAAGTAATGTCGATAAAGAGGAGG | 21 |
| | Reverse direction | GTGTCACCGTTGAAGAGAGTGG | 22 |
| CDK2 | Forward direction | ATGGATGCCTCTGCTCTCACTG | 23 |
| | Reverse direction | CCCGATGAGAATGGCAGAAAGC | 24 |

As a result, mRNA levels were significantly decreased in IL-32θ(281T)-expressing (IL-32θ+) breast tumor tissues, except for IL-6 (FIG. 3).

### [Example 4]

### Effect of IL-32θ(281T) on expression of inflammatory factors

In order to determine the effect of IL-32θ(281T) on the levels of inflammatory factors in human breast cancer cells, MDA-MB-231 breast cancer cells transfected with EV and IL-32θ(281T) were used. Overexpressed cell lines were evaluated using lipofectamine (A of FIG. 4a). Morphological changes in IL-32θ(281T) expressing MDA-MB-231 cells are shown in [B of FIG. 4a].

Specifically, cell culture was performed using MDA-MB-231 (ATCC^{®}HTB-26^{™}, Manassas, VA, USA) in Dulbecco's Modified Eagle's Medium (DMEM; HyClone Laboratories, Logan, UT, USA). MDA-MB-468 cells (ATCC^{®} HTB-132^{™}) were cultured in RPMI-1640 (HyClone Laboratories). Both media were supplemented with 10% heat-inactivated fetal bovine serum (Gibco BRL Life Technologies, Rockville, MD, USA), 100 U/mL penicillin, and 100 µg/mL streptomycin at 37°C and 5% CO₂.

For generation of IL-32 overexpressing cells, breast cancer cells were transfected with pcDNA3.1(+)-6x Myc empty vector (EV) and pcDNA3.1(+)-6x Myc IL-32θ(281T) vector. The pcDNA3.1(+)-6x Myc IL-32θ(281T) vector was prepared from the wild-type Myc-IL-32θ vector using the Muta-Direct^{™}Site-Directed Mutagenesis Kit (iNtRON Biotechnology). Briefly, cells were seeded in 60 Pi dishes (3 × 10⁵ cells/well) and transfected with 3 µg of vector using Lipofectamine^{®}2000 (Invitrogen, Carlsbad, CA, USA). Afterwards, breast cancer cells were selected for 2 weeks using medium containing 900 µg/mL G-418 (Duchefa Biochemie BV, Haarlem, The Netherlands). Only G-418 resistant colonies were cultured.

For conditioned medium (CM), THP-1 cells were seeded in T-75 flasks (3 × 10⁶ cells) and stimulated with 100 nM PMA (Millipore Sigma, Burlington, MA) for 72 hours. After treatment with PMA for 24 hours, cells were incubated with 20 ng/mL of IL-4 (PeproTech, Philadelphia, PA) and IL-13 (PeproTech) for another 48 hours to induce M2-polarized THP-1 macrophages. Then, the cells were washed with PBS (phosphate-buffered saline), and new culture medium was added after 24 hours. CM was collected and centrifuged to remove remaining cells.

For Western blotting, whole cell lysates were prepared in radioimmunoprecipitation assay (RIPA) buffer (iNtRON Biotechnology) containing 1X complete protease inhibitor cocktail and 1X PhosSTOP (Roche Diagnostics, Mannheim, Germany). In order to obtain nuclear and cytoplasmic proteins, cells were collected and nuclear fractionated using the NE-PER kit (Thermo Fisher Scientific) according to the manufacturer's instructions. Samples were subjected to sodium dodecyl sulfate-polyacrylamide gel electrophoresis and then transferred to 0.2 µm polyvinylidene difluoride membranes (Amersham Biosciences, Amersham, UK). The membrane was hybridized with the appropriate primary antibody overnight at 4°C. Antibodies are listed in [Table 3]. Western blotting was performed using a chemiluminescence detection kit (Advanstar, Cleveland, OH, USA), and protein bands were captured using an EZ-capture MG protein imaging system (ATTO, Tokyo, Japan). Western blotting bands were quantified using Image J open-source software (National Institutes of Health, Bethesda, MD).

**[Table 3]**

| Antibody | Species | Dilution | Cat# | Supplier |
|---|---|---|---|---|
| Myc-tag | Human | 1:5000 | 05-724 | Millipore |
| β-catenin | Human | 1:5000 | C2206 | |
| p-FAK | Human | 1:5000 | 700255 | Invitrogen |
| PARP | Human | 1:5000 | 9542s | |
| p50 | Human | 1:5000 | 3035s | |
| p-PI3K | Human | 1:5000 | 4228s | |
| p-GSK3 | Human | 1:5000 | 9331s | Cell Signaling Technology |
| p-IκBα | Human | 1:5000 | 2859s | |
| IκBα | Human | 1:5000 | 4812s | |
| p-AKT | Human | 1:5000 | 9271s | |
| p21 | Human | 1:5000 | 2947T | |
| PCNA | Human | 1:5000 | 2856s | |
| CDK2 | Human | 1:5000 | 2546T | |
| COX-2 | Human | 1:5000 | AF4198 | R&D Systems |
| GAPDH | Human | 1:5000 | sc-47724 | Santa Cruz |
| p65 | Human | 1:5000 | sc-8008 | Biotechnology |
| α-tubulin | Human | 1:5000 | sc-5286 | |
| Fibronectin | Human | 1:5000 | sc-9068 | |
| Vimentin | Human | 1:5000 | sc-66002 | |
| Cyclin A | Human | 1:5000 | sc-751 | |
| N-cadherin | Human | 1:5000 | ab18203 | Abcam |
| E-cadherin | Human | 1:5000 | ab1416 | |

For enzyme-linked immunosorbent assay (ELISA), MDA-MB-231 cells were cultured with CM for 24 hours, then, the culture medium was replaced with new culture medium and cultured for an additional 24 hours. Cell culture supernatants were collected and analyzed using ELISA kits (R&D systems) for human IL-1β, IL-6, IL-8 and PGE2 (GE Healthcare, Buckinghamshire, UK) according to the manufacturer's instructions.

As a result, RT-qPCR analysis showed that the mRNA levels of inflammatory factors were suppressed by IL-32θ(281T) in MDA-MB-231 cells (C in FIG. 4b). CM-treated MDA-MB-231 cells revealed that increased mRNA levels of inflammatory factors were suppressed by IL-32θ(281T) (D of FIG. 4b). The secreted protein levels of IL-1β, IL-6, IL-8 and prostaglandin E2 (PGE2) were determined by ELISA, and it was demonstrated that IL-32θ(281T) suppressed IL-1β, IL-6, IL-8 and PGE2 levels in CM-stimulated MDA-MB-231 cells (FIG. 5a). Western blot analysis after nuclear fractionation showed that IL-32θ(281T) inhibited NF-κB translocation to the nucleus in CM-stimulated MDA-MB-231 cells (E of FIG. 5b). Additionally, immunofluorescence (IF) analysis demonstrated that nuclear NF-κB was attenuated in IL-32θ(281T)-expressing MDA-MB-231 cells stimulated with CM (F of FIG. 5b).

### [Example 5]

### Modulatory effect of IL-32θ(281T) on migration of human breast cancer cells.

Inflammatory factors were found to be inhibited by IL-32θ(281T). The inventors of the present invention sought to determine whether IL-32θ(281T) affects the migration of breast cancer cells.

For immunofluorescence (IF) analysis, breast cancer cells were seeded on cell culture slides (SPL, Pocheon, Korea) and cultured overnight. Attached cells were fixed, permeabilized with paraformaldehyde and methanol, and blocked with 1% bovine serum albumin (BSA) in PBS at room temperature. Primary antibodies (1:200 diluted in 1% BSA) were added to the slides and incubated overnight at 4°C. After washing with PBS, slides were incubated with secondary antibodies (1:400 dilution). Antibodies are listed in [Table 3] above. Nuclear staining was performed by exposing cells to 4,6-diamidino-2-phenylindole (1:1,000 dilution in PBS) (Millipore Sigma) for 15 seconds. Cells were visualized using the EVOS M7000 Imaging System (Thermo Fisher Scientific).

For wound healing assay, breast cancer cells (1.5 × 10⁵ cells/well) were seeded in 24-well plates and cultured until 100% confluence. The plate was scraped using a sterile 200 µL pipette tip and washed with PBS to remove detached cells, and fresh serum-free medium was added after 24 hours. Plates were photographed under a microscope at 0 and 24 hours. Cell migration was measured using the scratch area with ImageJ.

Wound healing assays revealed a significant reduction in the migration of IL-32θ(281T)-expressing breast cancer cells compared to EV-transfected cells (FIGS. 6a, 6b). Additionally, Western blot analysis showed that IL-32θ(281T) suppressed mesenchymal markers (fibronectin, vimentin, N-cadherin) and upregulated epithelial marker E-cadherin in breast cancer cells (FIG. 7a). IF analysis showed that the fluorescence of E-cadherin was increased in IL-32θ(281T)-expressing cells, whereas the fluorescence of N-cadherin was decreased in IL-32θ(281T)-expressing cells (FIG. 7b).

### [Example 6]

### Intracellular signaling pathway of IL-32θ(281T) in human breast cancer cells.

The present invention shows that inflammatory factors and EMT markers are regulated by IL-32θ(281T). Additionally, it was revealed that NF-κB translocation was inhibited by IL-32θ(281T). The inventors of the present invention determined whether IL-32θ(281T) affects the intracellular signaling pathway of breast cancer cells.

Western blot analysis showed that IL-32θ(281T) inhibited p-FAK, p-PI3K, p-AKT and p-GSK3 compared to EV transfected breast cancer cells. Additionally, the degradation of β-catenin was induced by IL-32θ(281T) in breast cancer cells (FIG. 8).

### [Example 7]

### Inhibition of proliferation of human breast cancer cells through cell cycle arrest by IL-32θ(281T)

In the present invention, the FAK/PI3K/AKT pathway was found to be inhibited by IL-32θ(281T). In order to confirm the effect of IL-32θ(281T) on proliferation, MTS and BrdU assays were performed (FIG. 9A).

For the cell viability analysis, cell viability was assessed using Cell Titer 96^{®}One Solution Cell Proliferation Assay (Promega, Madison, WI, USA). Breast cancer cells (1 × 10⁴ cells/well) were seeded in 96-well plates and incubated every hour and washed with fresh medium. The cells were then incubated with 100 µL of 3-(4,5-dimethylthiazol-2-yl)-5-(3-carboxymethoxy phenyl)-2-(4-sulfophenyl)-2H-tetrazolium and the electronic coupling reagent phenazine methosulfate for 1 hour. Optical density (OD) was measured at 492 nm. Relative viability was quantified by normalization to control OD.

For the cell proliferation analysis, cell proliferation was determined based on BrdU (5'-bromo-2'-deoxyuridine) incorporation into DNA using Cell Proliferation Assay Kits (Cell Signaling Technology) according to the manufacturer's protocol. Breast cancer cells (5 × 10² cells/well) were seeded in 96-well plates, cultured for 72 hours, and labeled with BrdU for 24 hours. Cells were fixed, and detection antibodies and HRP-linked antibodies were used to detect BrdU. Color was developed using TMB (3,3',5,5'-tetramethylbenzidine), which is an HRP substrate, and OD was measured at 450 nm.

For the flow cytometry analysis, MDA-MB-231 (2 × 10⁵ cells/mL) and MDA-MB-468 (4 × 10⁵ cells/mL) cells were seeded in 60 pi dishes for 48 hours. Cells were then washed with PBS and fixed with 70% ethyl alcohol for 30 minutes. Cells were then washed with PBS and supplemented with PBS containing propidium iodide for 30 minutes. Fluorescence emission was measured and analyzed using a FACSCalibur flow cytometer (BD Biosciences, San Jose, CA, USA).

The cell viability and proliferation analysis indicated that IL-32θ(281T)-expressing breast cancer cells had significantly slower growth than EV transfected cells (FIG. 9a). Regulatory cell cycle (CDK2, cyclin A) and proliferating cell nuclear antigen (PCNA) proteins were inhibited by IL-32θ(281T) (C of FIG. 9a). Moreover, the antiproliferative protein p21 was upregulated by IL-32θ(281T) in breast cancer cells (C of FIG. 9a). In order to reveal the effect of IL-32θ(281T) on cell cycle arrest, PI staining was performed using flow cytometry. Flow cytometry results showed that the rate of S phase and G2/M phase arrest was higher in breast cancer cells expressing IL-32θ(281T) than in EV-transfected breast cancer cells (FIG. 9B).

### [Statistical Analysis]

Student's t-test was used to compare groups in the analysis of breast patient samples. One-way analysis of variance (ANOVA) with Student's t-test and Tukey's honest test was used to compare groups in *in vitro* experiments. Statistical analyzes were performed using GraphPad Prism software version 9.0. Results represent the mean ± SD of three experiments. All p-values were two-sided and were *p<0.05, **p<0.01, ***p<0.001 and ****p<0.0001.

### 2. Anti-inflammatory effect

### [Example 8]

### Expression and purification of recombinant human IL-32θ(A94V)

### <8-1> Expression of human IL-32θ (A94V)

A TEVSH vector was used to express IL-32θ(A94V), and recombinant IL-32θ(281T) DNA was inserted into the vector using Nde1 and Age1 restriction enzymes. The TEVSH vector includes a His tag for protein purification. A schematic diagram of the recombinant vector is shown in [FIG. 11].

Specifically, the IL-32θ(281T) coding sequence (SEQ ID NO: 3) was synthesized by Bioneer (Daejeon, Korea) using HT-oligo^{™} synthesis to improve protein expression efficiency. The synthesized DNA sequence was cloned into pTH24-based TEVSH vector (Addgene, Watertown, MA, USA) using Nde1 and Age1 restriction sites and a rapid DNA ligation kit (Thermo Fisher Scientific, Waltham, MA, USA). TEVSH was provided by Helena Berglund (Addgene plasmid #125194; http://n2t.net/addgene:125194; RRID:Addgene 125194) and confirmed by DNA sequencing (Bionics, Seoul, Korea). The recombinant TEVSH vector was transformed into DH5α by heat shock and purified using a mini prep kit (Intron Biotechnology, Sungnam, Korea). The IL-32θ(281T) expression vector was transformed into the Rosetta strain of *E. coli* by heat shock transformation. Successfully transformed single colonies were picked and cultured in Luria-Bertani (LB) medium containing ampicillin (100 µg/mL) at 37°C in a 200-rpm incubator for 4 hours. The cultured mixture was transferred to 2.4 L of fresh LB medium containing ampicillin (100 µg/mL) and grown in a 200-rpm incubator at 37°C until OD600 reached 0.6. IL-32θ(A94V) was induced by adding 0.5mM IPTG. Cells were grown in a 200-rpm incubator for 16 hours at 16°C.

### <8-2> Purification of IL-32θ (A94V) using Ni-NTA and CNBr-activated Sepharose 4B column

Serial purification was performed to improve the purity of IL-32θ(A94V). IL-32θ(A94V) was separated by binding to a Ni-NTA column and His tag, and the separated IL-32θ(281T) was purified once more using a CNBr-activated Sepharose 4B column combined with IL-32mAb KU32.

Specifically, after 16 hours, cells were harvested by centrifugation at 8,000 rpm for 10 minutes at 4°C. The supernatant was discarded, and the pellet was lysed using lysis buffer (50 mM Tris-HCl pH 8.0, 10% glycerol, 0.1% Triton X-100, 1X protease inhibitor cocktail, 2 mM MgCl2, 0.1mg lysozyme) and cultured on ice. The cells that were lysed for 30 minutes were sonicated for 1 minute (amplitude 35%, 10 seconds on, 10 seconds off) using a sonicator (Sonics & Materials, Inc., Newtown, CT, USA). The lysed cells were centrifuged at 13,000 rpm for 30 minutes at 4°C, and the supernatant was collected. Ni-NTA resin (Thermo Fisher Scientific) was loaded onto a PD-10 column and balanced with equilibration buffer (20 mM Tris-HCl pH 8.0, 200 mM NaCl). The lysate was mixed with 10 mL of equilibration buffer, loaded onto the column and washed with wash buffer (20 mM Tris-HCl pH 8.0, 200 mM NaCl, 25 mM imidazole). After washing, the elution buffer (20 mM Tris-HCl pH 8.0, 500 mM NaCl, 500mM imidazole) was loaded on the column, and the flow-through buffer containing IL-32θ was loaded on a CNBr-activated Sepharose 4B column (Sigma-Aldrich, St. Louis, MO, United States) and reloaded onto an IL-32mAb (KU32-52)-coupled CNBr-activated Sepharose 4B column prepared using the monoclonal antibody IL-32 mAb KU32-52. After washing several times with Tris (pH 8.0), IL-32θ(A94V) was eluted with 100 mM glycine (pH 3.0). The tube receiving the eluted solution contained 1M Tris (pH 8.0), which was one-tenth of the total volume. Purified IL-32θ (A94V) was dialyzed three times for 2 hours each with PBS (phosphate-buffered saline) using a spectra/Por membrane (Spectrum Laboratories, Piscataway, NJ, USA). The absence of endotoxin was assessed using polymyxin B (Sigma-Aldrich).

Purified IL-32θ(A94V) was analyzed using SDS-PAGE (FIG. 12A) and Western blot (FIGS. 12B, 11C). In SDS-PAGE analysis, no other notable protein bands were detected in the eluate from the Ni-NTA column except for IL-32θ (A94V), but several other proteins were observed through Western blotting. These off-target proteins were removed by further purification using a CNBr-activated Sepharose 4B column coupled to KU32-52. In the end, highly pure IL-32θ(A94V) protein was obtained with almost no other proteins.

### [Example 9]

### Binding of IL-32θ(A94V) to integrins αVβ3 and αVβ6.

The binding of IL-32θ(A94V) to integrins was predicted using the HDOCK server. In addition to structure-based binding predictions, the inventors of the present invention also performed IL-32θ(A94V)-integrin binding analysis.

Specifically, the tertiary structure of IL-32θ(A94V) was analyzed by I-TASSER (Iterative Threading ASSEmbly Refinement), which is a hierarchical approach to protein structure prediction and structure-based functional annotation. I-TASSER identifies structural templates with full-length models constructed from template-based fragment assembly simulations. The 3D model is then re-threaded through a protein function database to derive functional insights for the target. Protein-protein docking prediction was performed using the model with the highest reliability among the models derived from I-TASSER (34-36). Integrin-IL-32θ(A94V) binding was analyzed using the HDOCK server (http://hdock.phys.hust.edu.cn), which is a protein-protein binding prediction tool. HDOCK uses a hybrid docking strategy to predict binding complexes between two proteins. The extracellular segment structures of integrins αVβ3 (PDB ID: 1JV2) and αVβ6 (PDB ID: 5FFG) were provided in the Protein Data Bank (PDB).

IL-32θ(A94V) is shown as a yellow molecule including a helical structure, and the extracellular domains of integrins αVβ3 and αVβ6 provided by PDB are shown as orange molecules. The binding score of IL-32θ(A94V)-integrin αVβ3 was - 304.56, and the reliability score was 0.9565. The binding score of IL-32θ(A94V)-integrin αVβ6 was -358.20, and the confidence score was 0.9847, which was higher than that of IL-32θ(A94V)-integrin αVβ3 (FIG. 13). These results suggest that IL-32θ(A94V) has binding potential with these integrins and a stronger interaction with integrin αVβ6 than with integrin αVβ3.

For the IL-32θ(A94V)-integrin binding assay, a 96-well plate was coated with integrins αVβ3 and αVβ6, and then, IL-32θ(A94V) was added at the concentration shown in [FIG. 14a]. Cyclo-RGDfV, which is known to bind to the RGD binding site of integrin, was pretreated in a 96-well plate, and PBS was used as a control group. Additionally, wells were coated with integrins αVβ3 and αVβ6. IL-32θ(A94V) was treated at the concentrations shown in [FIG. 14b]. In other groups, medium including 10% FBS was pretreated in 96-well plates. Then, integrins αVβ3 and αVβ6 were added, and the control group was pretreated with serum-free medium. IL-32θ(A94V) was treated at the concentrations shown in [FIG. 14c].

Specifically, MaxiSorp flat bottom 96-well plates (Nunc, Roskilde, Denmark) were coated with 1 µg/mL of recombinant αVβ3 and αVβ6 integrins (R&D Systems, Minneapolis, MN, USA) diluted in PBS and incubated overnight at 4°C. Next, the wells were blocked with 1% BSA (Invitrogen, Waltham, MA, USA) dissolved in PBS for 1 hour at 37°C and then washed three times with PBS including 0.05% Tween 20. The wells were incubated with cyclo-RGDfV peptide (Peptide Institute Inc, Osaka, Japan) or 10% fetal bovine serum (FBS) and the remainder with PBS for 1 hour at 37°C. Next, the wells were incubated for 1 hour at 37°C with or without various concentrations of mutant IL-32θ(A94V), 10 µM cyclo-(RGDfV) or 10% FBS diluted in PBS. The wells were washed three times with wash buffer and incubated with IL-32 mAb KU32-52 diluted in PBS at a concentration of 0.2 µg/mL for 1 hour at 25°C. After incubation, the wells were washed three times with wash buffer and incubated with mouse-IgGκ light chain binding protein conjugated to HRP (m-IgGκ BP-HRP) (BETHYL, Waltham, MA USA) for 1 hour at 25°C. Next, the wells were washed three times with washing buffer and incubated with TMB substrate at 25°C for 20 minutes, and the color development reaction was stopped with 2.5 N H₂SO₄. Absorbance/optical density was measured at 450 nm using a microplate reader (Apollo LB 9110, Berthold Technologies GmbH, Bad Wildbad, Germany).

In the presence of integrins, the absorbance increased in a concentration-dependent manner for IL-32θ(A94V), but not for BSA (FIG. 14a), indicating that IL-32θ(A94V) also binds to integrins αVβ3 and αVβ6 similar to other isoforms. For both integrins αVβ3 and αVβ6, integrin-IL-32θ(A94V) binding was not significantly reduced by cyclo(RGDfV) (FIG. 14b), which suggests that IL-32θ(A94V) binds to a non-RGD binding site. In contrast to cyclo-(RGDfV), integrin-IL-32θ(A94V) binding was significantly inhibited by FBS (FIG. 14c). These results suggest that, similar to other isoforms such as IL-32β, IL-32θ(A94V) binds to the non-RGD binding sites of integrins αVβ3 and αVβ6, and these integrins may act as cell surface receptors for IL-32θ(A94V).

### [Example 10]

### Effect of IL-32θ(A94V) on the expressions of ICAM-1 and VCAM-1 in HUVEC cells stimulated with TNF-α.

In order to evaluate the effect of IL-32θ(A94V) on cells, the inventors of the present invention confirmed the expressions of integrins in HUVEC. THP-1 monocytes were used as a negative control group, not expressing integrins αVβ3 and αVβ6 on the surfaces thereof (FIG. 15A). In addition, the effect of IL-32θ(A94V) on the expressions of ICAM-1 and VCAM-1 in HUVEC cells stimulated with TNF-α was investigated. HUVECs were starved for 4 hours to minimize interference by FBS, pretreated with IL-32θ(A94V) for 1 hour, and stimulated with TNF-α for 4 or 6 hours. The mRNA levels of ICAM-1 and VCAM-1 were measured using RT-PCR (FIG. 15B) and RT-qPCR (FIG. 15C).

Specifically, HUVEC cells were cultured in Dulbecco modified Eagle medium (Welgene Incorporation, Daegu, Korea) supplemented with 10% (v/v) heat-inactivated fetal bovine serum (Hyclone Laboratories, Logan, UT, USA), penicillin (100 U/mL) and streptomycin (100 µg/mL). Cells were cultured in a chamber containing 5% CO₂ at 37°C.

Afterwards, for RNA extraction and RT-PCR, HUVEC cells (3 × 10⁵ cells/well) were cultured in a 6-well plate for 24 hours and starved in serum-free medium for 4 hours. Subsequently, cells were pretreated with IL-32θ(A94V) (100 ng/mL) for 1 hour and treated with TNF-α (10 ng/mL) for additional 4 hours. The treated cells were collected and lysed using the easy-BLUE^{™} Total RNA Extraction Kit (iNtRon Biotechnology, Seoul, South Korea) according to the manufacturer's instructions. For reverse transcription (RT) polymerase chain reaction (PCR), RNA (1 µg) was reverse transcribed into cDNA using oligo(dT) primers and M-MuLV reverse transcriptase (New England Biolabs, Ipswich, MA, USA). The synthesized cDNA was amplified using PCR Thermal Cycler Dice equipment (Takara, Otsu, Shiga, Japan). The following primer sets were used: Integrin αV, 5'-AGGAGAAGGTGCCTACGAAGCT-3' (forward, SEQ ID NO: 25) and 5'-GCACAGGAAAGTCTTGCTAAGGC-3' (reverse, SEQ ID NO: 26); Integrin β3, 5'-CATGGATTCCAGCAATGTCCTCC-3' (forward, SEQ ID NO: 27) and 5'-TTGAGGCAGGTGGCATTGAAGG-3' (reverse, SEQ ID NO: 28); Integrin β6, 5'-TCTCCTGCGTGAGACACAAGG-3' (forward, SEQ ID NO: 29) and 5'-GAGCACTCCATCTTCAGAGACG-3' (reverse, SEQ ID NO: 30); ICAM-1, 5'-AGCGGCTGACGTGTGCAGTAAT-3' (forward, SEQ ID NO: 31) and 5'-TCTGAGACCTCTGGCTTCGTCA-3' (reverse, SEQ ID NO: 32); VCAM-1, 5'-GATTCTGTGCCCACAGTAAGGC-3' (forward, SEQ ID NO: 33) and 5'-TGGTCACAGAGC CACCTTCTTG-3' (reverse, SEQ ID NO: 34); glyceraldehyde 3-phosphate dehydrogenase (GAPDH), 5'-AGAACATCATCCCTGCCTCT-3' (forward, SEQ ID NO: 5) and 5'-CTGCTTCACCACCTTCTTGA-3' (reverse, SEQ ID NO: 6). GAPDH was used as an internal control. PCR products were separated on a 2% agarose gel.

For RT-qPCR, HUVECs were obtained after treatment with or without IL-32θ(A94V) and TNF-α. mRNA extraction and cDNA synthesis were performed as previously described. RT-PCR was performed with a relative quantification protocol using Rotor-Gene 6000 series software 1.7 (Qiagen, Hilden, Germany) and Sensi FAST^{™} SYBR NO-ROX Kit (BIOLINE, London, UK). The expressions of all target genes were normalized to the expression of the housekeeping gene GAPDH. Each sample was ICAM-1, 5'-AGCGGCTGACGTGTGCAGTAAT-3' (forward, SEQ ID NO: 31), 5'-TCTGAGACCTCTGGCTTCGTCA-3' (reverse, SEQ ID NO: 32); VCAM-1, 5'-GATTCTGTGCCCACAGTAAGGC-3' (forward, SEQ ID NO: 33), 5'-TGGTCACAGAGCCACCTTCTTG-3' (reverse, SEQ ID NO: 34); GAPDH, 5'-AGAACATCATCCCTGCCTCT-3' (forward, SEQ ID NO: 5), 5'-CTGCTTCACCACCTTCTTGA-3' (reverse, SEQ ID NO: 6). GAPDH was used as an internal control. Using the comparative Ct method, the mRNA level of each gene was calculated relatively to the relative level of GAPDH, which was an internal reference.

For immunoblotting, HUVEC cells (3 × 10⁵ cells/well) were seeded in 6-well plates for 24 hours, pretreated with IL-32θ (A94V) (100 ng/mL) for 1 hour, and then treated with TNF-α (10 ng/mL). Cells were incubated at 4°C for 2 hours in a buffer including 50 mM Tris (pH 7.4), 150 mM NaCl, 1% NP-40, 0.1% sodium dodecyl sulfate (SDS), 0.25% sodium deoxycholate, 1 mM ethylene diamine tetraacetic acid (EDTA), 1 mM ethylene glycol tetraacetic acid, 1 mM orthovanadate, aprotinin (10µg/mL) and 0.4 mM phenyl methylsulfonyl fluoride (PMSF). The cells were lysed, and protein content was estimated using Bradford assay reagent (Bio-Rad Laboratories, Hercules, CA, USA) and UV spectrophotometry. Proteins (30 µg) were separated on a 10% SDS-polyacrylamide gel and transferred to PVDF membranes. Membranes were blocked with 5% skim milk for 1 hour at 25°C and incubated with primary antibodies against IL-32, His-tag (Sigma-Aldrich), FAK (Thermo Fisher Scientific), AKT (Cell Signaling, Danvers, MA, USA), JNK (Cell Signaling), IκB (Cell Signaling), p65 (Cell Signaling), p50 (Cell Signaling), PARP (Cell Signaling), c-Jun (Santa Cruz Biotechnology, Dallas, TX, USA) c-Fos (Santa Cruz Biotechnology) for 1 hour at 25°C. After incubation, the membranes were incubated with secondary antibody (anti-rabbit or anti-mouse IgG antibody) (BETHYL) for 1 hour at 25°C. Finally, protein bands were visualized using an enhanced chemiluminescence Western blotting detection kit (Advansta, San Jose, CA USA).

For immunofluorescence, HUVEC cells (1.0 × 10⁵ cells/well) were seeded in 8-well slide chambers for 24 hours, starved overnight and incubated with IL-32θ(A94V) (100 ng/mL) and TNF-α (10 ng/mL) for 6 hours. After treatment, cells were fixed using 4% paraformaldehyde for 10 minutes, then incubated with ice-cold methanol and blocked using 1% BSA in PBS for 1 hour at 25°C. The cells were then incubated overnight at 4°C with primary antibodies against ICAM-1 and VCAM-1 (Beijing Solarbio Science & Technology Co., Beijing, China), and the cells were incubated with secondary antibodies conjugated to Cy3 (Merck Millipore, Darmstadt, Germany). After 1 hour at 25°C, DAPI staining was performed. Two washing steps with PBS were performed between each step. Afterwards, the cells were mounted in mounting buffer (Sigma-Aldrich) and observed under a fluorescence microscope.

As a result, the mRNA expression levels of ICAM-1 and VCAM-1 were increased by TNF-α and significantly decreased by IL-32θ(A94V). Protein expression was also analyzed using immunofluorescence. The fluorescence intensity of ICAM-1 and VCAM-1 increased upon TNF-α stimulation as expected and was suppressed by IL-32θ(A94V) (FIG. 16).

### [Example 11]

### Effect of IL-32θ(A94V) on monocyte-endothelial cell adhesion

ICAM-1 and VCAM-1, which are expressed in endothelial cells stimulated by proinflammatory cytokines such as TNF-α, play an important role in monocyte-endothelial cell adhesion, which is a step in vascular inflammation.

For monocyte-endothelial cell adhesion assay, HUVEC cells (1.5 × 10⁴ cells/well) were seeded in 4-well slide chambers for 24 hours, starved overnight and incubated with IL-32θ(A94V) (100 ng/mL). After pretreatment for 6 hours, the cells were treated with TNF-α (10ng/mL). THP-1 cells were labeled with 5 µM calcein-AM (Molecular Probes, Eugene, OR, USA) for 30 minutes in RPMI-1640 without FBS. THP-1 cells labeled with calcein-AM were added to 4-well slide chambers containing HUVECs and cultured in RPMI-1640 including 10% FBS for 30 minutes. Subsequently, unbound monocytes were removed by washing three times with PBS. The remaining monocytes were determined using fluorescence microscopy. Fluorescence intensity was measured using ImageJ software version 1.5.

As a result, IL-32θ(A94V) inhibited the expression of monocyte-endothelial cell adhesion molecules in endothelial cells, thereby reducing monocyte-endothelial cell adhesion (FIGS. 15 to 17). The adhesion of calcein-AM-labeled THP-1 (green) to HUVEC was increased by TNF-α in co-cultures of HUVEC and THP-1 cells. Adhesion between HUVEC and THP-1 cells was significantly reduced by IL-32θ(A94V) (FIG. 17A). Fluorescence was measured using Image J software (FIG. 17B).

### [Example 12]

### Effect of IL-32θ(A94V) on phosphorylation levels of FAK, AKT and JNK in HUVEC cells stimulated with TNF-α

The inventors of the present invention revealed that IL-32θ(A94V) inhibits the expressions of ICAM-1 and VCAM-1 in HUVECs stimulated with TNF-α. The inventors of the present invention performed Western blot to identify the intracellular signaling pathway underlying the regulation of the expressions of ICAM-1 and VCAM-1 mediated by IL-32θ (A94V).

As a result, IL-32θ(A94V) significantly reduced the phosphorylation level of TNF-α-induced focal adhesion kinase (FAK), which is a well-known integrin-mediated signaling molecule that is an upstream molecule of protein kinase B (AKT) and c-Jun. N-terminal kinase (JNK) and IL-32θ (A94V) also downregulated the phosphorylation levels of AKT and JNK by inhibiting FAK as expected (FIG. 18).

### [Example 13]

### Effect of IL-32θ(A94V) on nuclear translocation of NF-κB and AP-1 in HUVEC cells stimulated with TNF-α.

TNF-α stimulation activates the FAK/AKT signaling pathway, thereby inducing the nuclear translocation of NF-κB (p65/p50) through phosphorylation of IκB. Additionally, the activation of JNK accelerates the nuclear translocation of AP-1 (c-fos/c-jun). These transcription factors promote the expressions of ICAM-1 and VCAM-1. The inventors of the present invention determined the phosphorylation levels of IκB and nuclear translocation of NF-κB (p65/p50) and AP-1 (c-Fos/c-Jun).

In order to prepare cytoplasmic and nuclear extracts, HUVEC cells (2.5 × 10⁵ cells/well) were seeded in cell culture dishes for 24 hours and pretreated with IL-32θ(A94V) (100 ng/mL) for 1 hour, and then, the cells were treated with TNF-α (10 ng/mL) for 30 minutes before harvesting and fractionation using NE-PER nuclear and cytoplasmic extraction reagent (Thermo Fisher Scientific) according to the manufacturer's instructions. Equal amounts of proteins from this extract (50 µg) were separated by SDS-polyacrylamide gel electrophoresis and transferred to PVDF membranes. Subsequent steps in the procedure were followed as described for Western blotting above. PARP was used as a nuclear protein marker.

As a result, IL-32θ(A94V) slightly attenuated TNF-α-induced IκB phosphorylation (FIG. 19A). Nuclear translocation of AP-1 (c-Fos/c-Jun) and NF-κB (p65/p50) was increased by TNF-α stimulation and inhibited by IL-32θ(A94V) (FIG. 19B). These results show that IL-32θ(A94V) regulates the nuclear translocation of AP-1 (c-Fos/c-Jun) and NF-κB (p65/p50) in HUVECs stimulated with TNF-α, and as a result, the expressions of ICAM- 1 and VCAM-1 are attenuated.

### Industrial Applicability

Since the interleukin-32θ mutant (IL-32θ mutant, IL-32θ(281T)) of the present invention can control the progression of cancer, especially breast cancer, by suppressing the expression of inflammatory factors by regulating the NF-κB pathway and suppressing EMT markers and cell cycle-related factors through the FAK/PI3K/AKT pathway, it has industrial applicability. In addition, since IL-32θ(281T) can provide anti-inflammatory effects in chronic inflammatory diseases such as arteriosclerosis by weakening monocyte-endothelial adhesion by binding to cell surface receptors, integrin αVβ3 and αVβ6, and inhibiting the expression of ICAM-1 and VCAM-1 in TNF-α-stimulated HUVECs, it has industrial applicability. In addition, since it can also be utilized as a research material for various cancers and/or inflammatory diseases, it has industrial applicability.

### Sequence List Free Text

SEQ ID NO: 1 represents an amino acid sequence of IL-32θ (A94V).
SEQ ID NO: 2 represents a nucleotide sequence of IL-32θ(281T).
SEQ ID NO: 3 represents a nucleotide sequence of IL-32θ (281T) for increasing protein expression efficiency.
SEQ ID NO: 4 represents a wild-type sequence of IL-32θ(281T).
SEQ ID NO: 5 represents a forward primer sequence for GAPDH.
SEQ ID NO: 6 represents a reverse primer sequence for GAPDH.
SEQ ID NO: 7 represents a forward primer sequence for IL-32θ.
SEQ ID NO: 8 represents a reverse primer sequence for IL-32θ.
SEQ ID NO: 9 represents a forward primer sequence for IL-1β.
SEQ ID NO: 10 represents a reverse primer sequence for IL-1β.
SEQ ID NO: 11 represents a forward primer sequence for IL-6.
SEQ ID NO: 12 represents a reverse primer sequence for IL-6.
SEQ ID NO: 13 represents a forward primer sequence for IL-8.
SEQ ID NO: 14 represents a reverse primer sequence for IL-8.
SEQ ID NO: 15 represents a forward primer sequence for COX-2.
SEQ ID NO: 16 represents a reverse primer sequence for COX-2.
SEQ ID NO: 17 represents a forward primer sequence for N-cadherin.
SEQ ID NO: 18 represents a reverse primer sequence for N-cadherin.
SEQ ID NO: 19 represents a forward primer sequence for Vimentin.
SEQ ID NO: 20 represents a reverse primer sequence for Vimentin.
SEQ ID NO: 21 represents a forward primer sequence for PCNA.
SEQ ID NO: 22 represents a reverse primer sequence for PCNA.
SEQ ID NO: 23 represents a forward primer sequence for CDK2.
SEQ ID NO: 24 represents a reverse primer sequence for CDK2.
SEQ ID NO: 25 represents a forward primer sequence for integrin αV.
SEQ ID NO: 26 represents a reverse primer sequence for integrin αV.
SEQ ID NO: 27 represents a forward primer sequence for integrin β3.
SEQ ID NO: 28 represents a reverse primer sequence for integrin β3.
SEQ ID NO: 29 represents a forward primer sequence for integrin β6.
SEQ ID NO: 30 represents a reverse primer sequence for integrin β6.
SEQ ID NO: 31 represents a forward primer sequence for ICAM-1.
SEQ ID NO: 32 represents a reverse primer sequence for ICAM-1.
SEQ ID NO: 33 represents a forward primer sequence for VCAM-1.
SEQ ID NO: 34 represents a reverse primer sequence for VCAM-1.

## Claims

1. A pharmaceutical composition for preventing or treating cancer, comprising an interleukin-32θ mutant (IL-32θ mutant).

2. The pharmaceutical composition of claim 1, wherein the interleukin-32θ mutant comprises an amino acid sequence of SEQ ID NO: 1.

3. The pharmaceutical composition of claim 2, wherein the interleukin-32θ mutant is encoded by a nucleotide sequence of SEQ ID NO: 2.

4. The pharmaceutical composition of claim 2, wherein the interleukin-32θ mutant is encoded by a nucleotide sequence of SEQ ID NO: 3.

5. The pharmaceutical composition of claim 1, wherein the cancer is at least any one selected from the group consisting of stomach cancer, lung cancer, liver cancer, pancreatic cancer, colon cancer, prostate cancer and breast cancer.

6. A pharmaceutical composition for preventing or treating an inflammatory disease, comprising an interleukin-32θ mutant (IL-32θ mutant).

7. The pharmaceutical composition of claim 6, wherein the inflammatory disease is at least any one selected from the group consisting of arthritis, rheumatoid arthritis, gout, hepatitis, asthma, keratitis, gastritis, enteritis, nephritis, tuberculosis, bronchitis, pleurisy, peritonitis, spondylitis, pancreatitis, inflammatory pain, urethritis, cystitis, arteriosclerosis, sepsis, dermatitis, periodontitis and gingivitis.

8. An anti-cancer immunotherapeutic composition, comprising an interleukin-32θ mutant (IL-32θ mutant).

9. An anti-inflammatory immunotherapeutic composition, comprising an interleukin-32θ mutant (IL-32θ mutant).

10. A recombinant vector for expressing an interleukin-32θ mutant (IL-32θ mutant), comprising a nucleotide sequence of SEQ ID NO: 3.

11. Use of an interleukin-32θ mutant (IL-32θ mutant) for use in the treatment of cancer.

12. Use of an interleukin-32θ mutant (IL-32θ mutant) for use in the treatment of an inflammatory disease.

13. A method for treating cancer, comprising administering a composition comprising an interleukin-32θ mutant (IL-32θ mutant) to a cancer patient.

14. A method for treating an inflammatory disease, comprising administering a composition comprising an interleukin-32θ mutant (IL-32θ mutant) to a patient with an inflammatory disease.
